# EUROPEAN PATENT APPLICATION

(11) **EP 4 360 711 A1**
(43) Date of publication of application: **01.05.2024**
(21) Application number: 22204042.0
(22) Date of filing: 27.10.2022
(51) Int. Cl.: A61P 35/00, A61K 48/00, A61K 31/711, A61K 38/17

(54) **TREATMENT OF PROSTATE CANCER BY PROMOTING PDE4D7**

(71) Applicant: Koninklijke Philips N.V., 5656 AG Eindhoven (NL)
(72) Inventor: HOFFMANN, Ralf Dieter, Eindhoven (NL); BAILLIE, George, Eindhoven (NL)
(74) Representative: Philips Intellectual Property & Standards

(57) **Abstract**

The invention relates to a product for use in the treatment, prevention or amelioration of prostate cancer in a subject in need thereof, wherein the product induces the expression of PDE4D7 or promotes activity of phosphodiesterase 4D7. The product may be a direct or indirect activator of PDE4D7 activity or a product for expressing or promting expression of PDE4D7 an a subject. The invention further defines a kit of parts and an in vitro or ex vivo use of the product.

## Description

### FIELD OF THE INVENTION

The invention relates to a product for use in the treatment, prevention or amelioration of prostate cancer in a subject in need thereof. In particular the product is used to induce the expression of PDE4D7 or promote activity of phosphodiesterase 4D7.

### BACKGROUND OF THE INVENTION

Cancer is a class of diseases in which a group of cells display uncontrolled growth, invasion and sometimes metastasis. These three malignant properties of cancers differentiate them from benign tumors, which are self-limited, do not invade or metastasize. Among men, the three most commonly diagnosed cancers are prostate, lung and colorectal cancer in developed countries. Particularly prostate cancer is the most common malignancy in European males. In 2002 in Europe, an estimated 225,000 men were newly diagnosed with prostate cancer and about 83,000 died from this disease.

Prostate cancer, for example, is traditionally diagnosed via the serum level of prostate-specific antigen (PSA). However, PSA is not prostate cancer-specific and can be raised in other circumstances, leading to a large number of false-positives (cancer is not found in around 70% of men with raised PSA levels who undergo biopsy). Furthermore, there will be an unpredictable number of false-negatives who later develop prostate cancer in the presence of a "normal" PSA test.

In patients who undergo treatment, the most important clinical prognostic indicators of disease outcome are the stage, pretherapy PSA level, and Gleason score. The higher the grade and the stage, the poorer the prognosis. Nomograms can be used to calculate the estimated risk of the individual patient. The predictions are based on the experience of large groups of patients. [250] A complicating factor is that the majority of patients have multiple independent tumor foci upon diagnosis, and these foci have independent genetic changes and molecular features. Because of this extensive inter-focal heterogeneity, it is a risk that the prognostication is set based on the wrong tumor focus.

Androgen ablation therapy causes remission in 80-90% of patients undergoing therapy, resulting in a median progression-free survival of 12 to 33 months. After remission, an androgen-independent phenotype typically emerges, wherein the median overall survival is 23-37 months from the time of initiation of androgen ablation therapy. How androgen-independence is established and how it reestablishes progression is unclear. Androgen ablation therapy may for example include taking antiandrogen drugs.

Therefore there is a constant need for new and improved methods for treatment of cancers such as prostate cancer. Moreover, methods for reducing or preventing resistance to treatments such as antiandrogen drugs or PARP inhibitors.

These drawbacks, among others, are overcome by the products and methods as outlined in the appended claims.

### SUMMARY OF THE INVENTION

In a first aspect the invention relates to a product for use in the treatment, prevention or amelioration of prostate cancer in a subject in need thereof, wherein the product induces the expression of PDE4D7 or promotes activity of phosphodiesterase 4D7.

In a second aspect the invention relates to a kit of parts comprising a product as defined in the first apsect of the invention.

In a third aspect the invention relates to an ex vivo or in vitro use of a product as defined in in the first apsect of the invention to increase expression of PDE4D7 and/or activity of phosphodiesterase 4D7 in a cell.

### BRIEF DESCRIPTION OF THE DRAWINGS

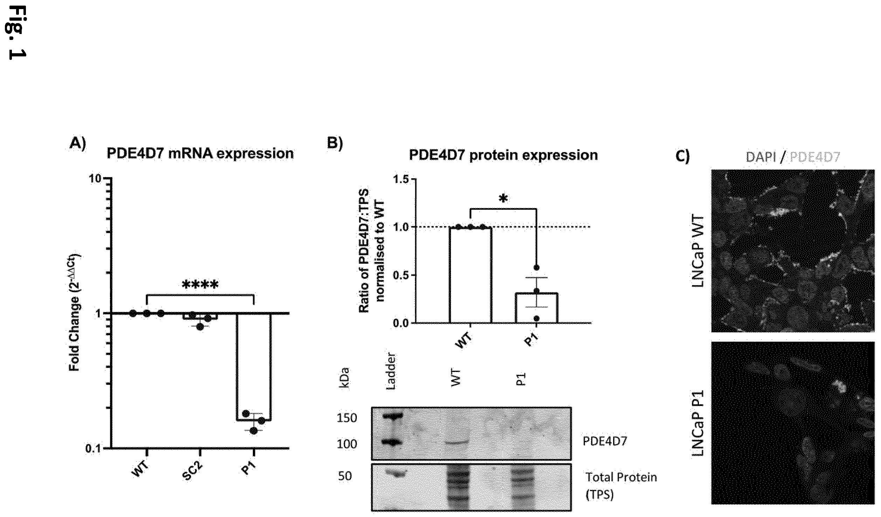

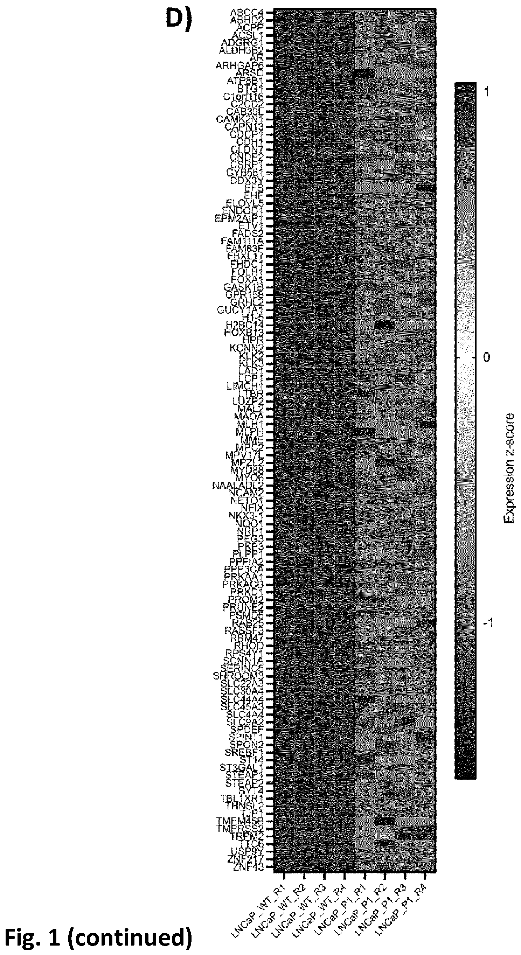

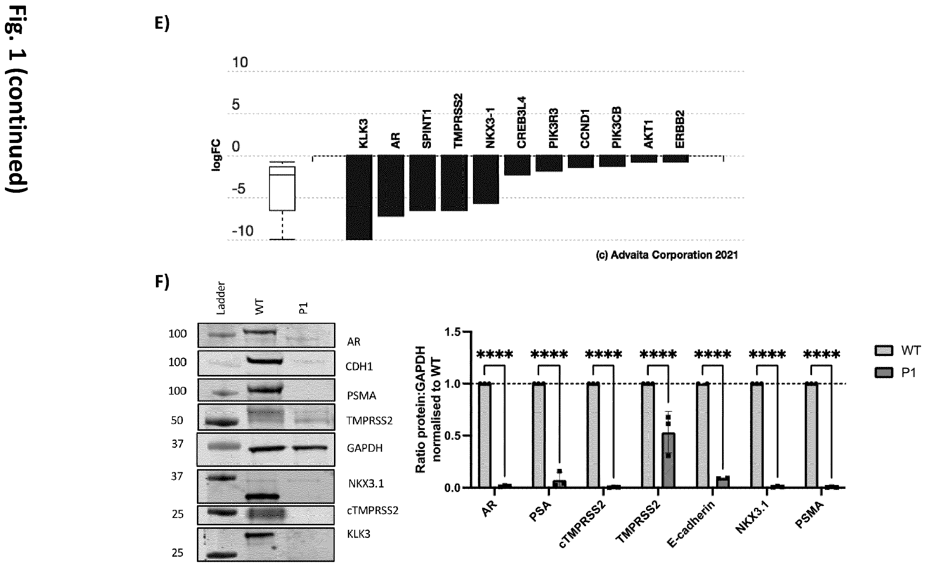

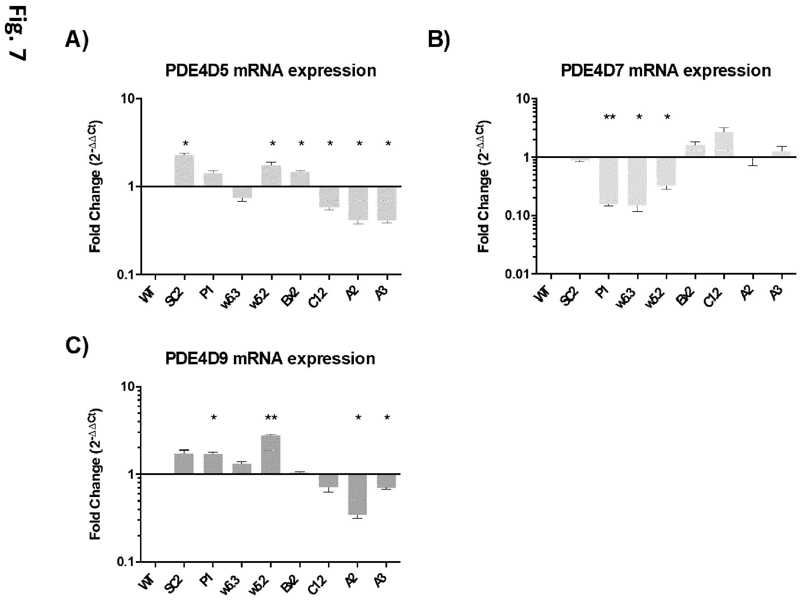

Fig. 1: Generation of PDE4D7-knockdown LNCaP cell lines and differences in PCa-related genes. A) PDE4D7 mRNA expression via qRT-PCR. Fold change relative to LNCaP WT (2-ΔΔCt) for shRNA-scrambled (SC2) and shRNA-PDE4D7 knock-down (P1) B) PDE4D7 protein expression in WT and shRNA-PDE4D7 knock-down (P1) LNCaPs. C) Immunocytochemistry staining of PDE4D7 protein expression (yellow) in WT and P1 LNCaP. D) Heatmap of z-scores of top differentially expressed genes between WT and P1 LNCaPs via RNAseq. E) Log fold change (FC) of top altered genes in PCa-related pathways. F) Western blot and quantification of protein expression of PCa-related RNA-seq-identified genes in WT and P1 LNCaPs. Error bars represent mean +/- SEM, N=3, ^{∗} p<0.05, ^{∗∗∗∗} p<0.0001.

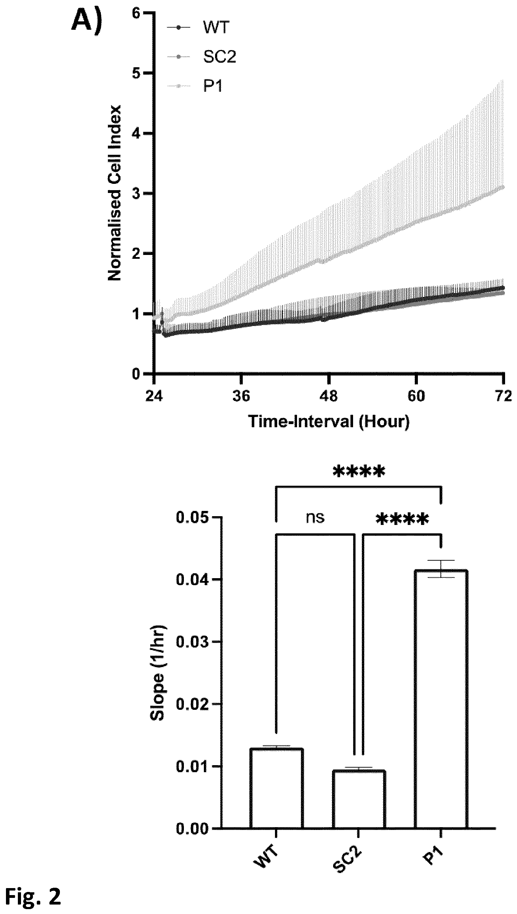

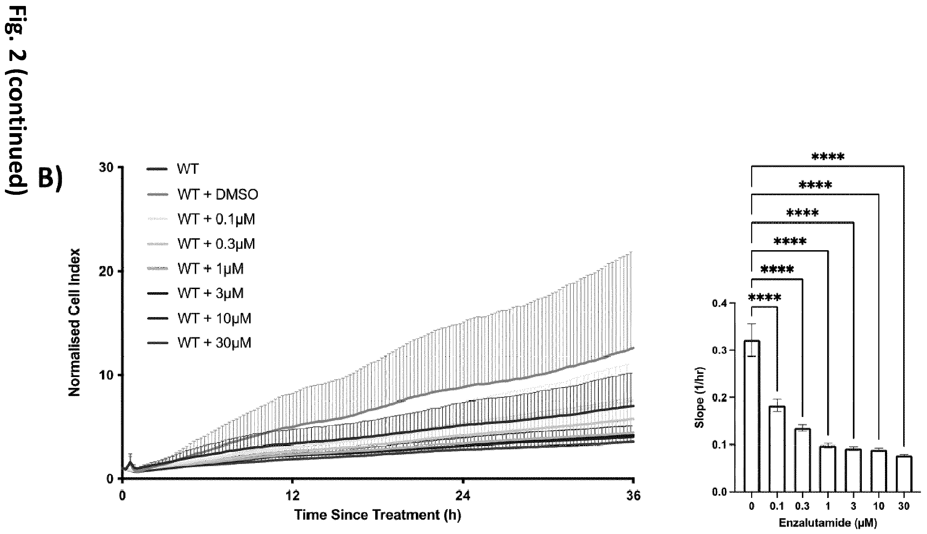

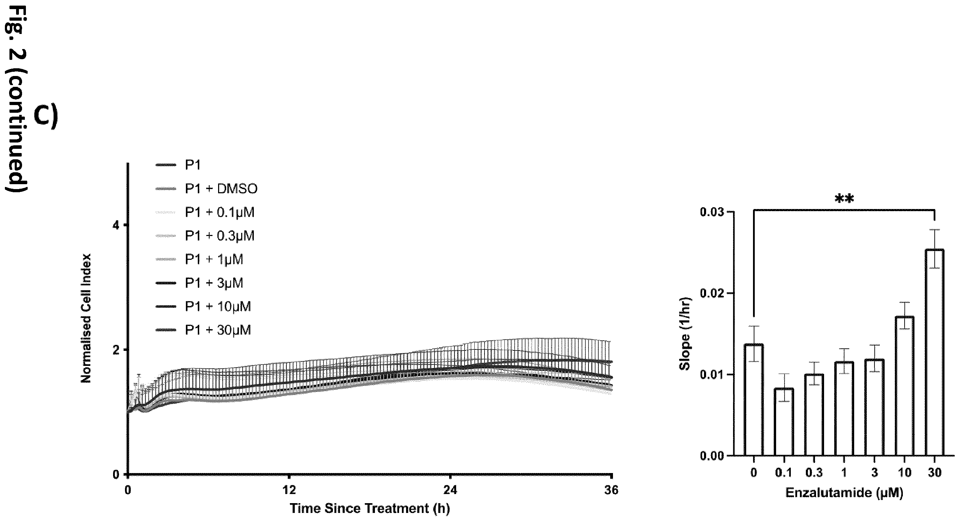

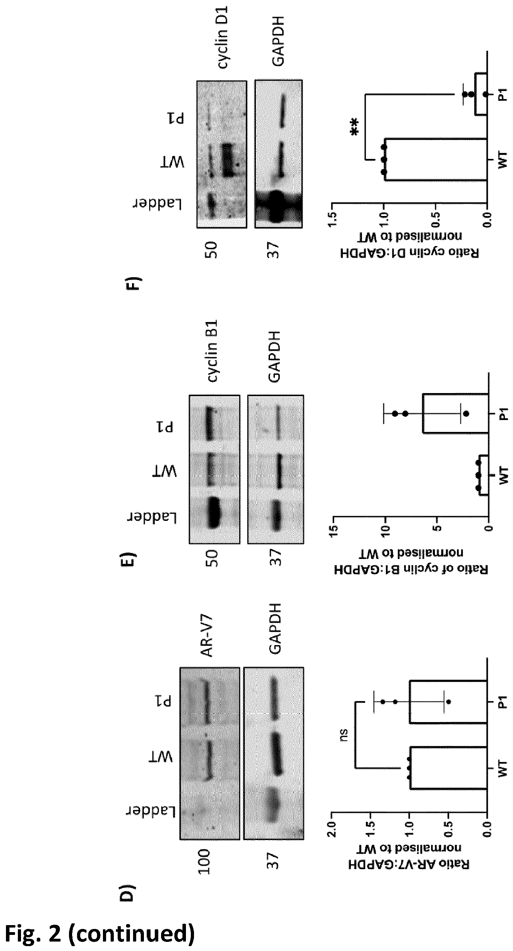

Fig. 2: PDE4D7 knockdown in LNCaP cells enhances growth and confers resistance to enzalutamide. A) Real-time proliferation of LNCaP WT, shRNA-scrambled (SC2) and shRNA-PDE4D7 knock-down (P1) cells. Slope analysis via linear regression. B & C Real-time analysis of LNCaP WT (B) and shRNA-PDE4D7 knock-down (P1) cells (C) with enzalutamide (0µM - 30µM) or 0.1% DMSO. D) AR-V7 protein expression western blot and quantification in WT and P1 LNCaPs. E) Cyclin B1 and F) Cyclin D1 protein expression western blot and quantification in WT and P1 LNCaPs. Error bars represent mean +/- SEM, N=3, ^{∗∗} p<0.01, ^{∗∗∗∗} p<0.0001).

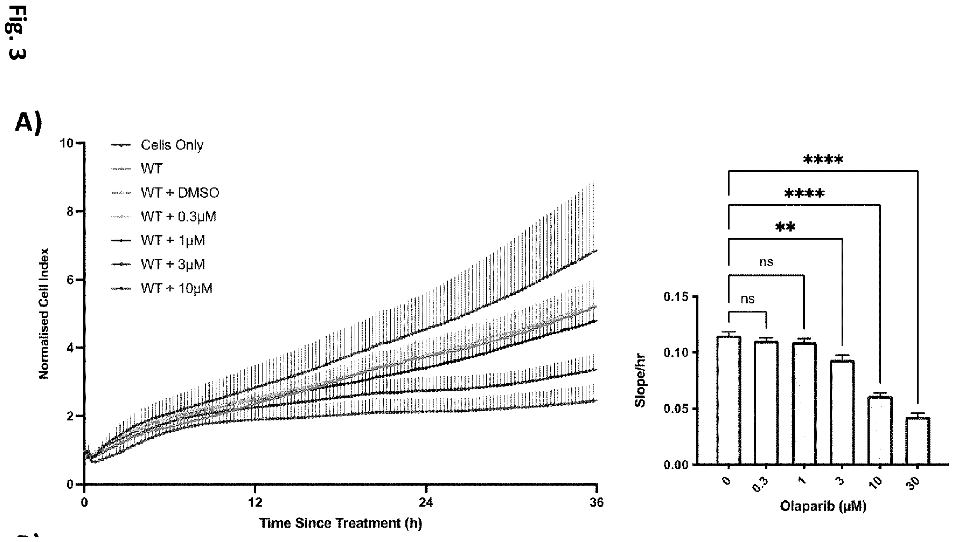

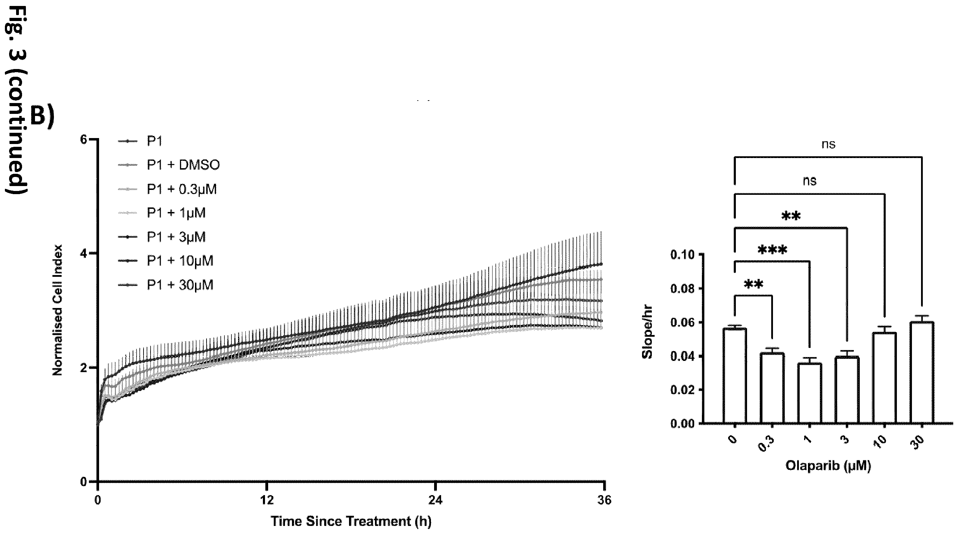

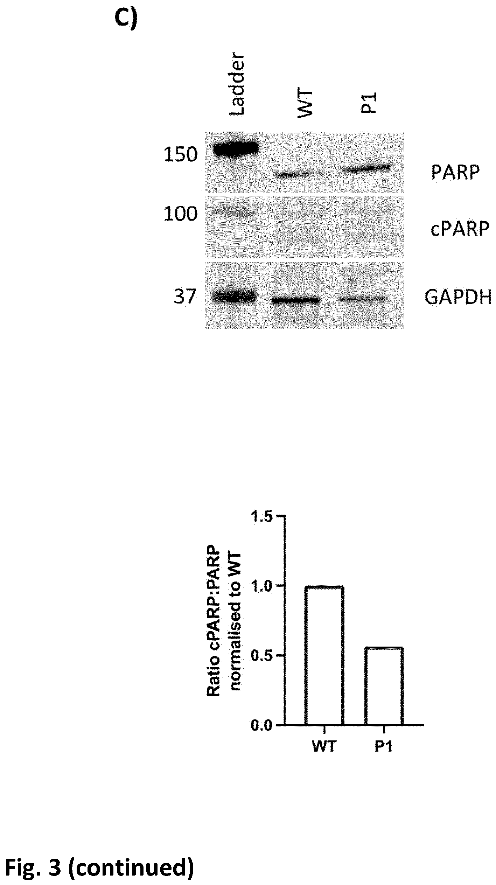

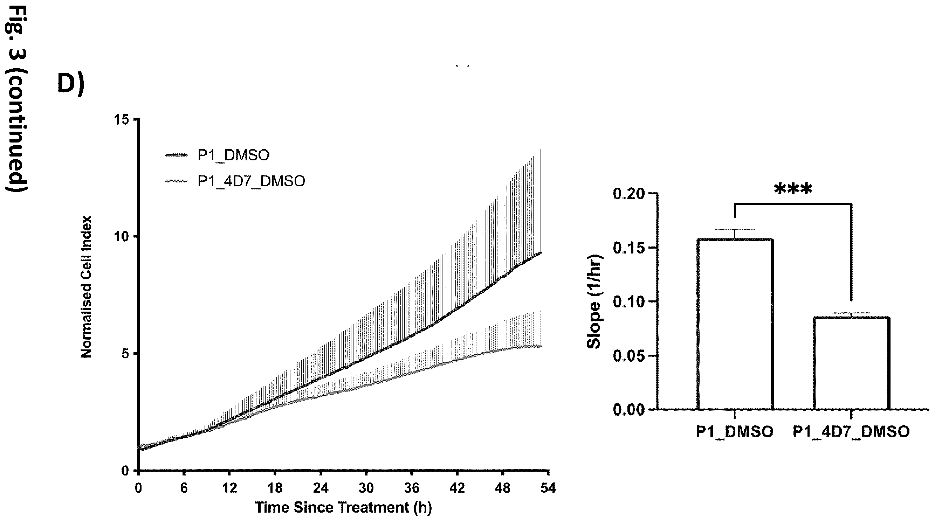

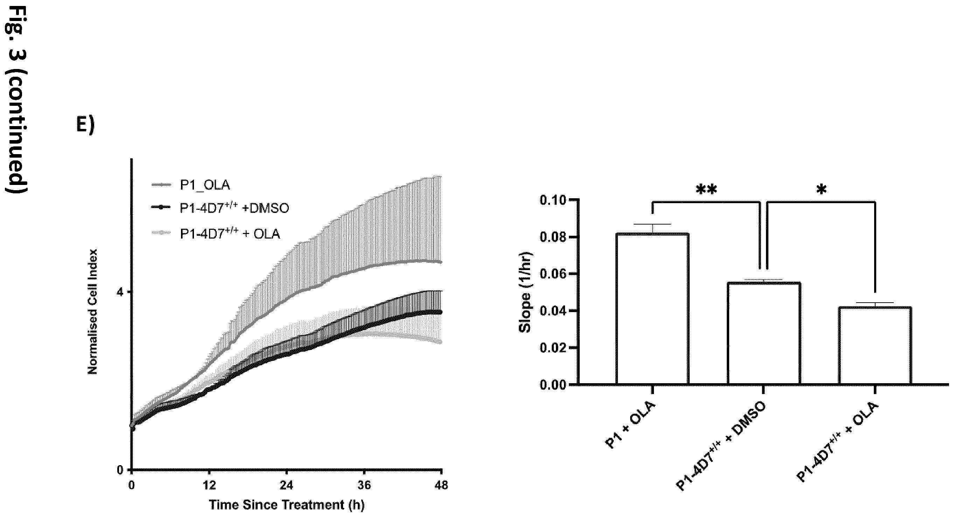

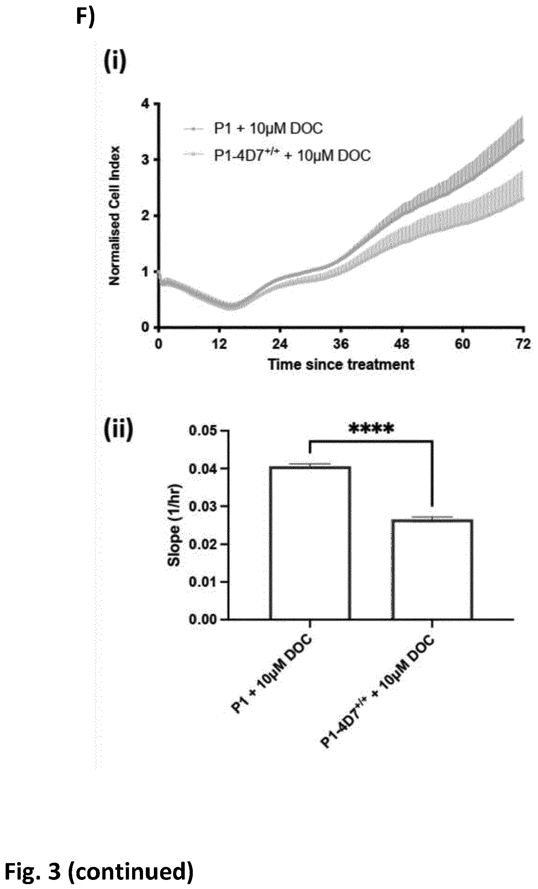

Fig. 3: PDE4D7-knockdown in LNCaP cells enhances resistance to olaparib. A & B) Real-time proliferation of LNCaP WT (A) and shRNA-PDE4D7 knock-down (P1) cells following treatment with olaparib (0µM - 30µM) or 0.1% DMSO. Normalised cell index to timepoint of treatment. Slope analysis via linear regression between 0-30h. C) Protein expression of PARP and cleaved PARP (cPARP) western blot and quantification between WT and P1 LNCaPs. D) Real-time proliferation of P1 and LNCaP P1-PDE4D7+/+ transient re-expression cells in response to 0.1% DMSO (D) or 10µM Olaparib (E) or 10 µM Docetaxel (F). Normalised cell index to timepoint of treatment. Slope analysis between 0-48h post-treatment. Error bars represent mean ± SEM, N=3, ^{∗} p<0.05, ^{∗∗} p<0.01, ^{∗∗∗} p<0.001, ^{∗∗∗∗} p<0.0001.

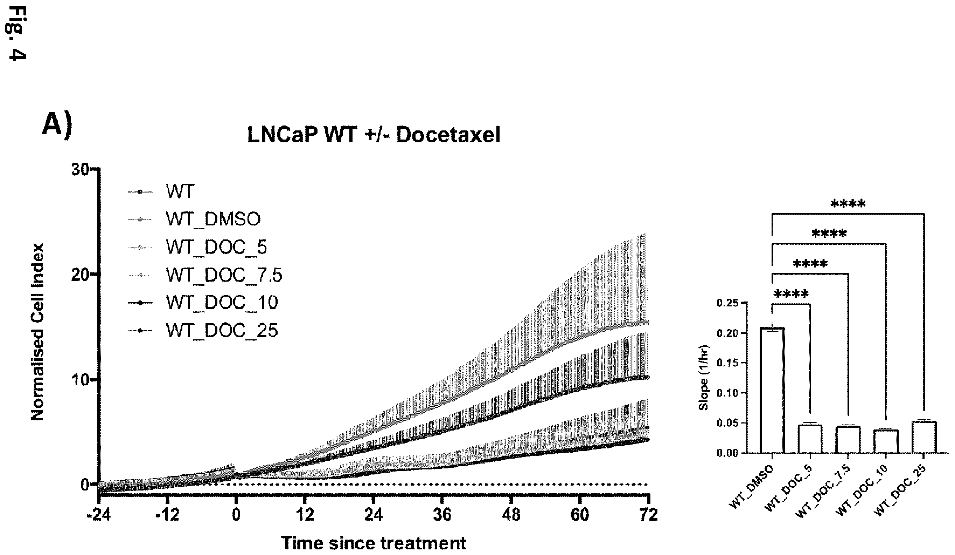

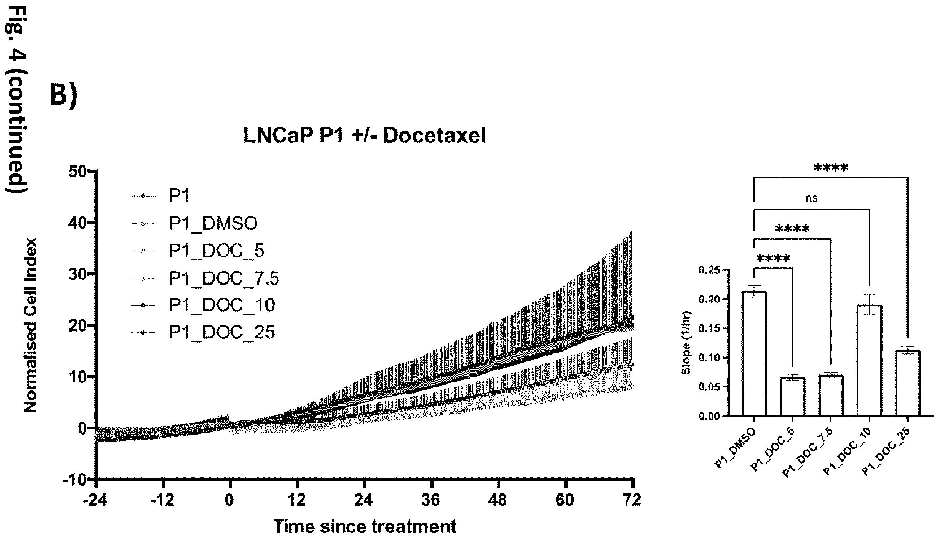

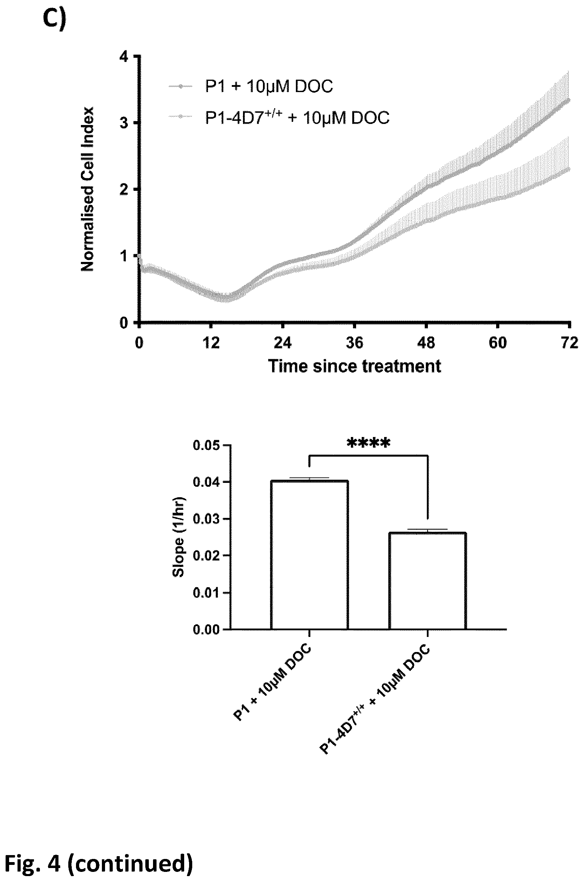

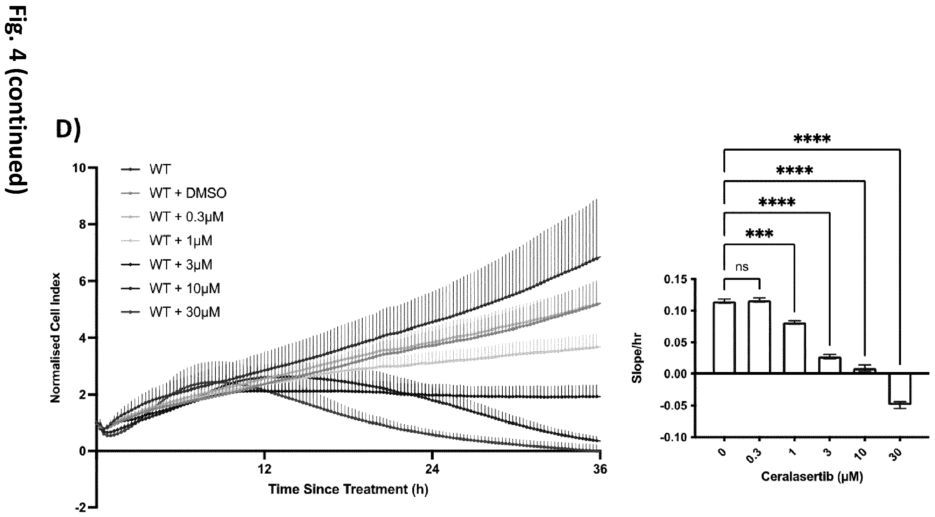

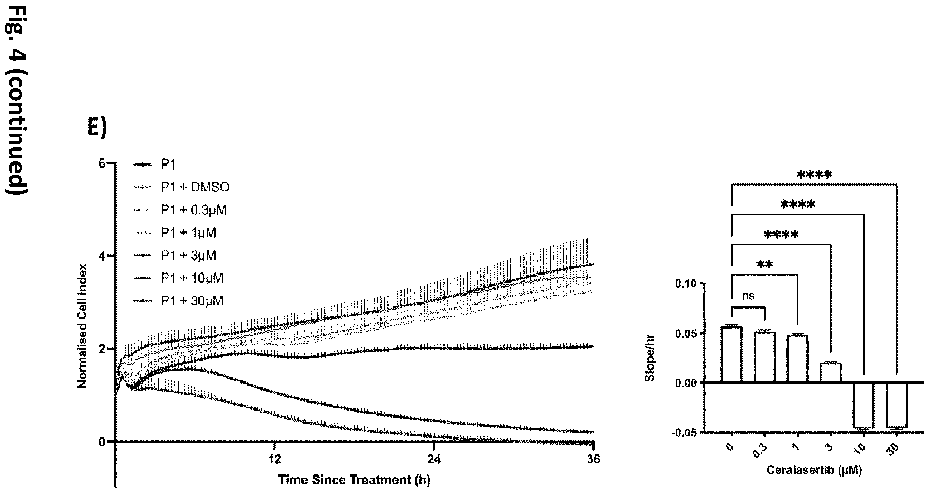

Fig. 4: DNA damage induction and repair agents affect both WT and PDE4D7-knockdown LNCaPs (P1). Real-time growth analysis of docetaxel (DMSO: control (0.1%); DOC_5: 5 µM docetaxel; DOC_7.5: 7.5 µM docetaxel; DOC_10: 10 µM docetaxel; DOC_25: 25 µM docetaxel; treatment on WT (A) or P1 (B) LNCaPs, or P1 and (C) P1-PDE4D7+/+ (after transient PDE4D7 re-expression) LNCaPs including the slope analysis 0-48h since treatment. D & E) Real-time proliferation of LNCaP WT (D) and P1 (E) cells with ceralasertib (0µM - 30µM). Slope analysis 0-30h post-treatment. Normalised cell index to timepoint of treatment. Error bars represent mean +/- SEM, N=3, ^{∗} p<0.05, ^{∗∗} p<0.01, ^{∗∗∗} p<0.001, ^{∗∗∗∗} p<0.0001.

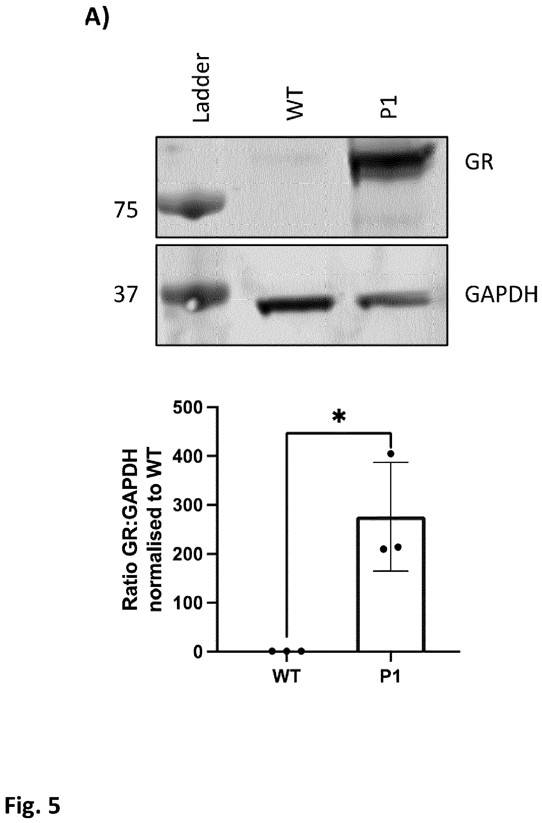

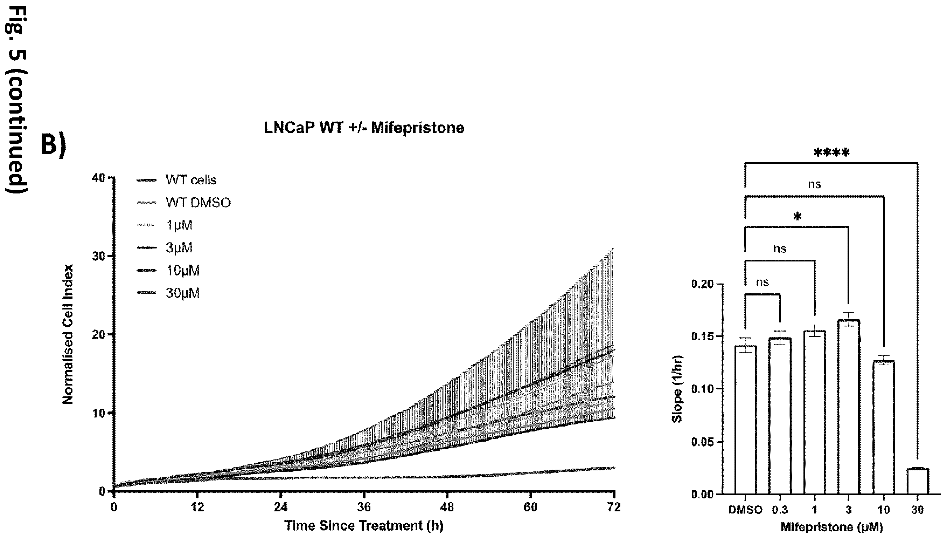

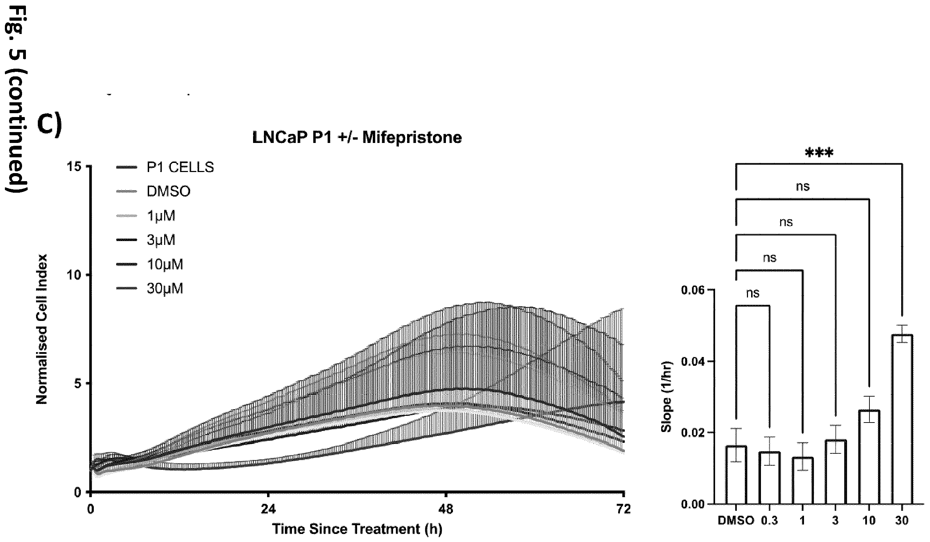

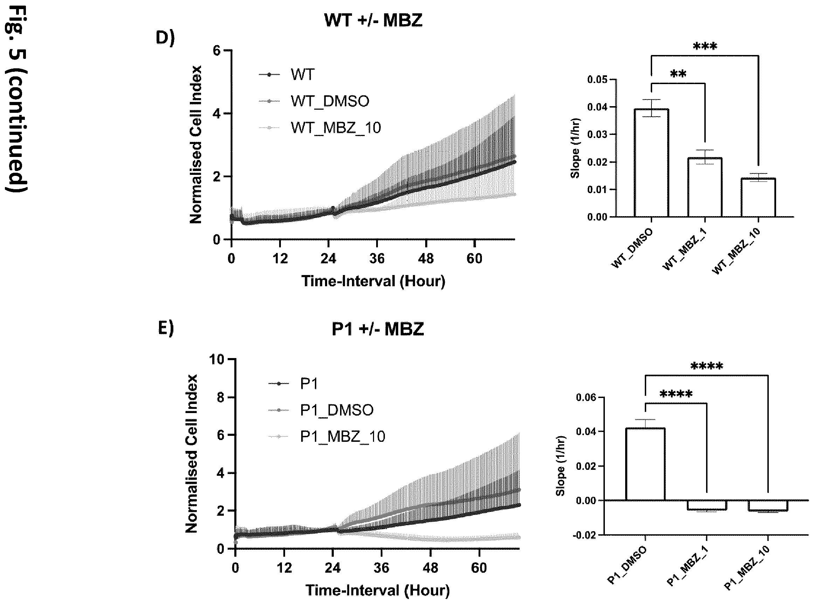

Fig. 5: PDE4D7-knockdown alters LNCaP response to various compounds. A) Glucocorticoid receptor (GR) protein expression western blot and quantification between WT and PDE4D7-knock-down P1 LNCaPs. B & C) Real-time proliferation of WT (B) and P1 (C) LNCaPs upon treatment with 0-30µM mifepristone or 0.1% DMSO (control) includingslope analysis 0-48h post-treatment. D & E) Real-time proliferation of WT (D) and PDE4D7-knock-down P1 (E) LNCaPs upon treatment with 1-10µM mebendazole or 0.1% DMSO including slope analysis 0-48h post-treatment. Error bars represent mean +/- SEM, N=3, ^{∗} p<0.05, ^{∗∗} p<0.01, ^{∗∗∗} p<0.001, ^{∗∗∗∗} p<0.0001.
A & B) Real-time growth analysis of WT (A) and PDE4D7-knock-down P1 (B) cells upon treatment with metformin (0-25µM) including slope analysis from 0-48h post-treatment. C & D) Real-time growth analysis of WT (C) and PDE4D7-knock-down P1 (D) cells upon treatment with PDE4 activator ML-R2 (1-10µM) including slope analysis from 0-48 post-treatment. Error bars represent mean +/- SEM, N=3, ^{∗∗} p<0.01, ^{∗∗∗} p<0.001, ^{∗∗∗∗} p<0.0001.Fig. 7: Difference in PDE4D isoform mRNA expression between knockdown LNCaP cell lines. RT-qPCR of various PDE4D knockdown LNCaP cell lines shows fold changes in gene expression relative to WT (2-ΔΔCt) for PDE4D5 (A), PDE4D7 (B) and PDE4D9 (C.) One-way ANOVA was performed on ΔΔCt values. Error bars represent mean ± SEM for N=3 (^{∗} = p<0.05, ^{∗∗} =p<0.01, ^{∗∗∗} =p<0.001).

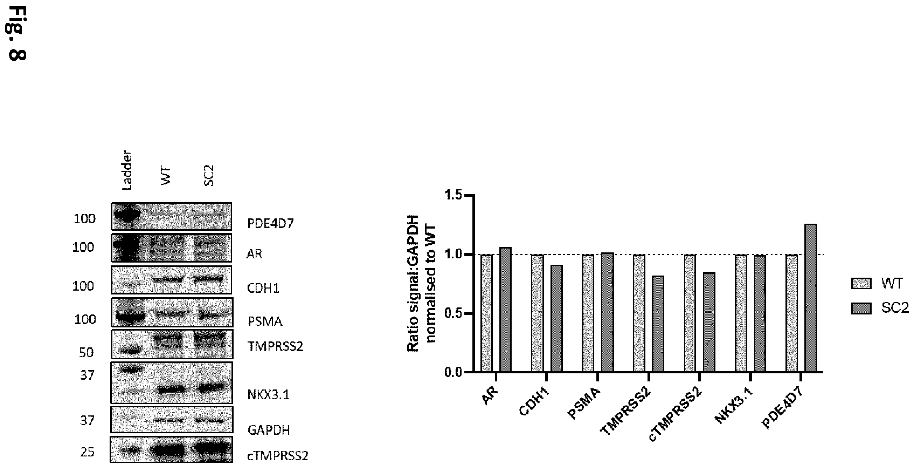

Fig 8: RNA-seq identified genomic alterations between WT and scrambled-shRNA (SC2) LNCaPs. Western blot and quantification of protein expression of PCa-related RNA-seq genes in WT and SC2 LNCaPs (N=1).

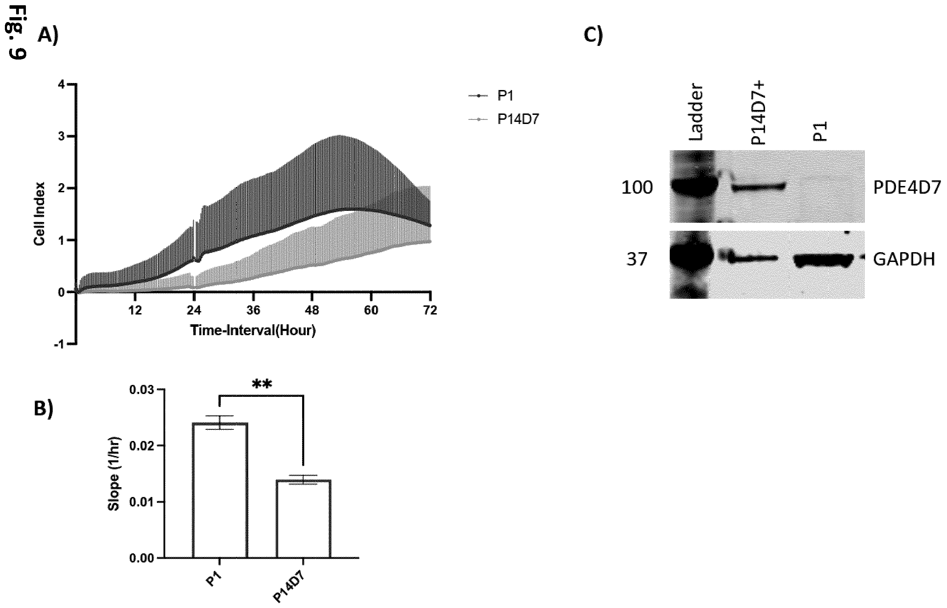

Fig. 9: Re-expression of PDE4D7 reduces growth. (A) Real-time growth analysis of PDE4D7-knowdown P1 and PDE4D7-knowdown with stable re-introduction of PDE4D7 P1-4D7+ cells. (B) Slope analysis from 0-72 post-onset. (C) Western blot of protein expression of PDE4D7 isoform in P1-4D7+ and P1 cells. Error bars represent mean ± SEM for N=3, ^{∗∗} p<0.01, ^{∗∗∗} p<0.001, ^{∗∗∗∗} p<0.0001.

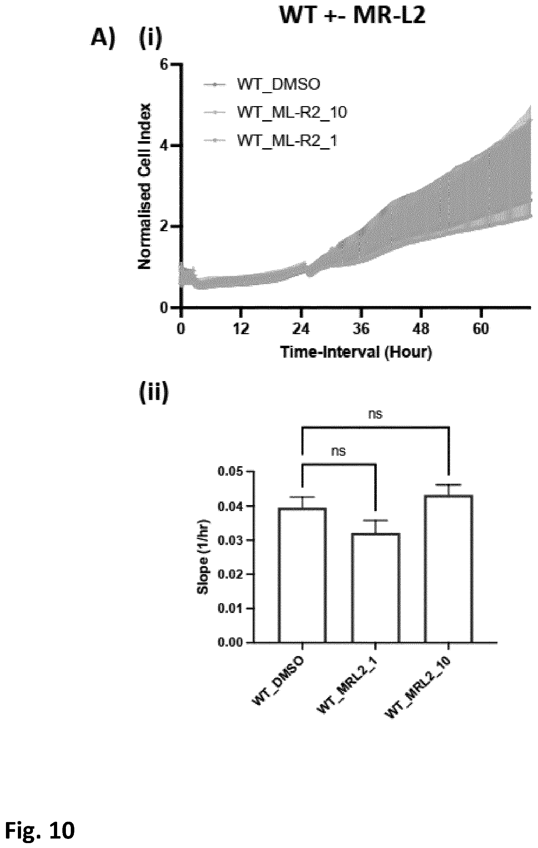

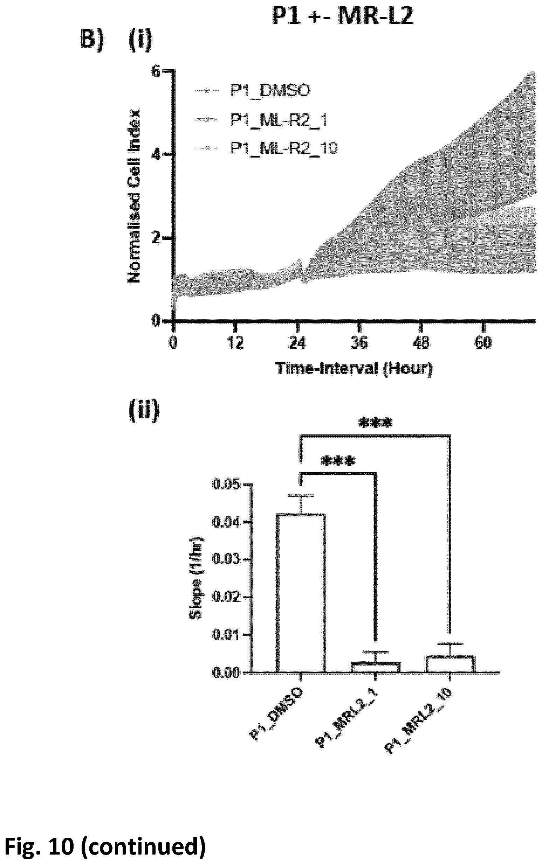

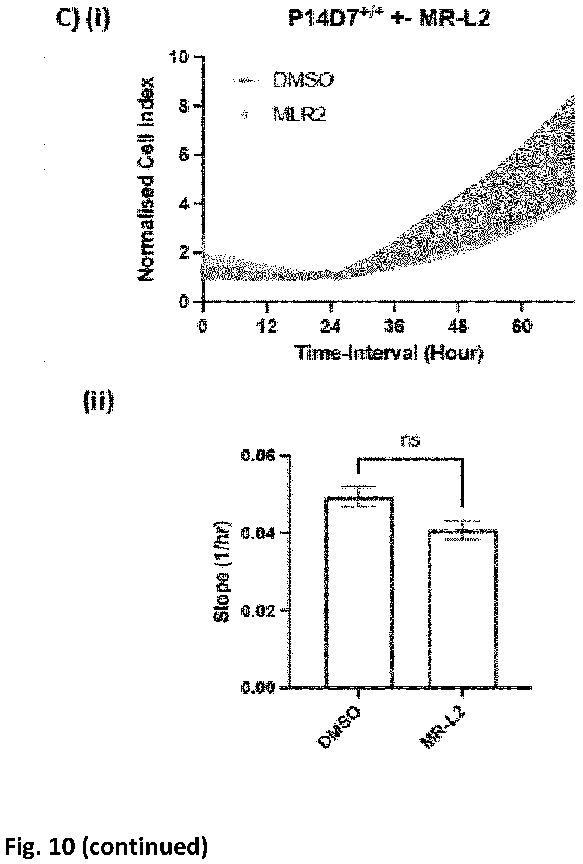

Fig. 10: Testing of the PDE4 activating compound MR-L2 on the real-time proliferation of prostate cancer cells; (i) real-time proliferation; (ii) slope analysis of real-time proliferation. A) WT_DMSO: LNCaP wild-type DMSO control (0.1%); WT_ML-R2_1: LNCaP wild-type with 1 µM ML-R2; WT_ML-R2_10: LNCaP wild-type with 10 µM ML-R2; B) P1_DMSO: LNCaP PDE4D7 knockdown cell line P1 DMSO control (0.1%); P1_ML-R2_1: LNCaP PDE4D7 knockdown cell line P1 with 1 µM ML-R2; P1_ML-R2_10: LNCaP PDE4D7 knockdown cell line P1 with 10 µM ML-R2; C) P14D7+/+_DMSO: LNCaP PDE4D7 knockdown cell line P1 with transient re-expression of PDE4D7 DMSO control (0.1%); P14D7+/+DMSO: LNCaP PDE4D7 knockdown cell line P1 with transient re-expression of PDE4D7 and with ML-R2 (10 µM). Error bars represent mean ± SEM for N=3, ^{∗∗} p<0.01, ^{∗∗∗} p<0.001, ^{∗∗∗∗} p<0.0001.

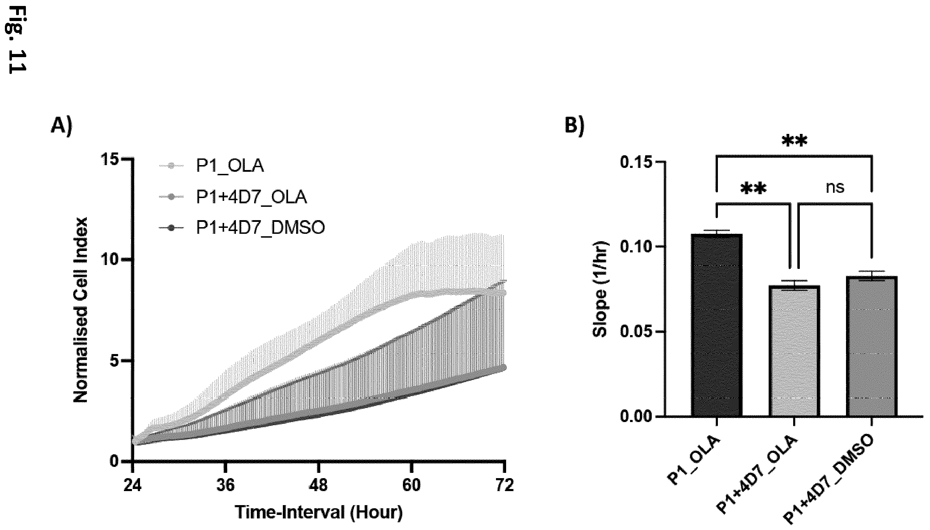

Fig. 11: Testing of the PARP inhibitor olaparib on the real-time proliferation of prostate cancer cells. A) P1_OLA: LNCaP PDE4D7 knockdown cell line P1 with 10 µM olaparib; P1+4D7_OLA: LNCaP PDE4D7 knockdown cell line P1 with transient re-expression of PDE4D7 and with 10 µM olaparib; P1+4D7 DMSO: LNCaP PDE4D7 knockdown cell line P1 with transient re-expression of PDE4D7 DMSO control (0.1%); B) slope analysis of real-time proliferation. Error bars represent mean ± SEM for N=3, ^{∗∗} p<0.01, ^{∗∗∗} p<0.001, ^{∗∗∗∗} p<0.0001.

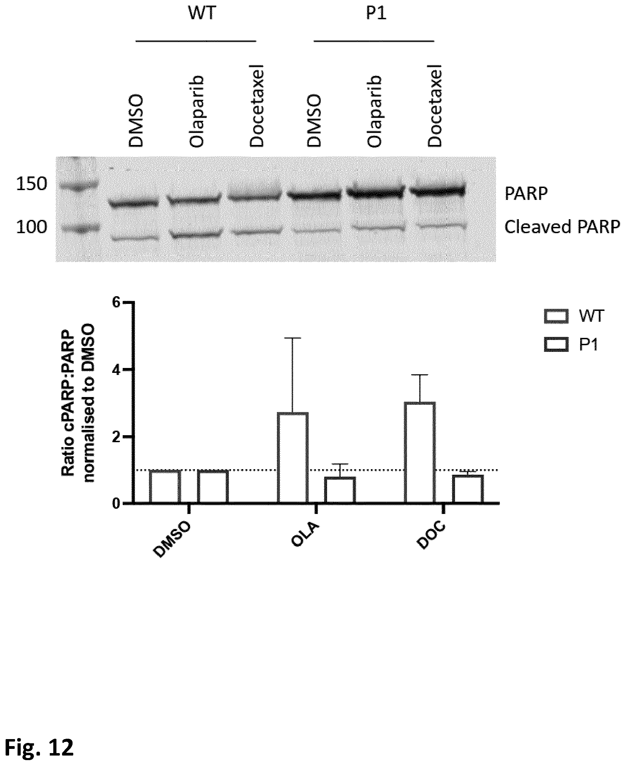

Fig. 12: Ratio of PARP and cleaved PARP (PARPc) by western blot analysis in LNCaP WT and LNCaP PDE4D7 knockdown cell line P1 upon incubation with 0.1% DMSO (control), 10 µM Olaparib, and 10 nM docetaxel including quantitation of western blot bands normalised to DMSO.

### Definitions

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20%, preferably ±15%, more preferably ±10%, and even more preferably ±5%.

**"About" and "approximately":** these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

**"Antagonist"** and **"inhibitor":** These terms are used interchangeably, and they refer to a compound or agent having the ability to reduce or inhibit a biological function of a target protein or polypeptide, such as by reducing or inhibiting the activity or expression of the target protein or polypeptide. Accordingly, the terms "antagonist" and "inhibitor" are defined in the context of the biological role of the target protein or polypeptide. An inhibitor need not completely abrogate the biological function of a target protein or polypeptide, and in some embodiments reduces the activity by at least 20%, 30%, 40%, 50%, 60%, 70%, 80%, 90%, 95%, or 99%. While some antagonists herein specifically interact with (e.g., bind to) the target, compounds that inhibit a biological activity of the target protein or polypeptide by interacting with other members of the signal transduction pathway of which the target protein or polypeptide are also specifically included within this definition. Non-limiting examples of biological activity inhibited by an antagonist include those associated with the development, growth, or spread of a tumor, or an undesired immune response as manifested in autoimmune disease.

**"Anti-cancer effect":** This refers to the effect a therapeutic agent has on cancer, e.g., a decrease in growth, viability, or both of a cancer cell. The IC50 of cancer cells can be used as a measure the anti-cancer effect. IC50 refers to a measure of the effectiveness of a therapeutic agent in inhibiting cancer cells by 50%.

**"Alleviating cancer":** The term, in the context of specific cancers and/or their pathologies, refers to degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

**"Compositions", "products" or "combinations":** These encompass those compositions suitable for various routes of administration, including, but not limited to, intravenous, subcutaneous, intradermal, subdermal, intranodal, intratumoral, intramuscular, intraperitoneal, oral, nasal, topical (including buccal and sublingual), rectal, vaginal, aerosol and/or parenteral or mucosal application. The compositions, formulations, and products according to the disclosure invention normally comprise the drugs/compound/inhibitor (alone or in combination) and one or more suitable pharmaceutically acceptable excipients or carriers.

**"Combination therapy", or "in combination with":** These term refers to the use of more than one compound or agent to treat a particular disorder or condition. For example, Compound 1 may be administered in combination with at least one additional therapeutic agent. By "in combination with," it is not intended to imply that the other therapy and Compound 1 must be administered at the same time and/or formulated for delivery together, although these methods of delivery are within the scope of this disclosure. Compound 1 can be administered concurrently with, prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after), one or more other additional agents. In general, each therapeutic agent will be administered at a dose and/or on a time schedule determined for that particular agent. The other therapeutic agent can be administered with Compound 1 herein in a single composition or separately in a different composition. Higher combinations, e.g., triple therapy, are also contemplated herein.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of" and "consisting of".

As used herein, the term "conventional techniques" refers to a situation wherein the methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the term "identity" refers to a measure of the identity of nucleotide sequences or amino acid sequences. In general, the sequences are aligned so that the highest order match is obtained. "Identity" per se has an art-recognized meaning and can be calculated using published techniques. See, e.g.: (Computational Molecular Biology, Lesk, A. M., ED., Oxford University Press, New York, 1988; Biocomputing: Informatics And Genome Projects, Smith, D. W., ED., Academic Press, New York, 1993; Computer Analysis Of Sequence Data, Part I, Griffin, A. M., And Griffin, H. G., EDS., Humana Press, New Jersey, 1994; Sequence Analysis In Molecular Biology, Von Heinje, G., Academic Press, 1987; and Sequence Analysis Primer; Gribskov, M. and Devereux, J., eds., M Stockton Press, New York, 1991). While there exist a number of methods to measure identity between two nucleotide sequences or amino acid sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in Guide To Huge Computers, Martin J. Bishop, ed., Academic Press, San Diego, 1994, and Carillo, H., and Lipton, D., Siam J. Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Molec. Biol. (1990) 215:403).

As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations per each 100 nucleotides of the reference amino acid sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: X is intended that the amino acid sequence of the polypeptide is identical to the reference sequence except that the amino acid sequence may include up to five amino acid alterations per each 100 amino acids of the reference amino acid of SEQ ID NO: X. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As used herein, the term "in vitro" refers to experimentation or measurements conducted using components of an organism that have been isolated from their natural conditions.

As used herein, the term "ex vivo" refers to experimentation or measurements done in or on tissue from an organism in an external environment with minimal alteration of natural condition.

As used herein, the term "nucleic acid", "nucleic acid molecule" and "polynucleotide" is intended to include DNA molecules and RNA molecules. A nucleic acid (molecule) may be single-stranded or double-stranded, but preferably is double-stranded DNA.

As used herein, the terms "sequence" when referring to nucleotides, or "nucleic acid sequence", "nucleotide sequence" or "polynucleotide sequence" refer to the order of nucleotides of, or within, a nucleic acid and/or polynucleotide. Within the context of the current invention a first nucleic acid sequence may be comprised within or overlap with a further nucleic acid sequence.

As used herein, the term "subject" or "individual" or "animal" or "patient" or "mammal," used interchangeably, refer to any subject, particularly a mammalian subject, for whom diagnosis, prognosis, or therapy is desired. Mammalian subjects include humans, domestic animals, farm animals, and zoo-, sports-, or pet-animals such as dogs, cats, guinea pigs, rabbits, rats, mice, horses, cattle, cows, bears, and so on. As defined herein a subject may be alive or dead. Samples can be taken from a subject post-mortem, i.e. after death, and/or samples can be taken from a living subject.

As used herein, terms "treatment", "treating", "palliating", "alleviating" or "ameliorating", used interchangeably, refer to an approach for obtaining beneficial or desired results including, but not limited to, therapeutic benefit. By therapeutic benefit is meant eradication or amelioration or reduction (or delay) of progress of the underlying disease being treated. Also, a therapeutic benefit is achieved with the eradication or amelioration or reduction (or delay) of progress of one or more of the physiological symptoms associated with the underlying disease such that an improvement or slowing down or reduction of decline is observed in the patient, notwithstanding that the patient can still be afflicted with the underlying disease.

### DETAILED DESCRIPTION OF EMBODIMENTS

The section headings as used herein are for organizational purposes only and are not to be construed as limiting the subject matter described.

A portion of this invention contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent invention, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention relates, unless otherwise indicated. Other specifically defined terms are to be construed in a manner consistent with the definition as provided herein. The preferred materials and methods are described herein, although any methods and materials similar or equivalent to those described herein can be used in the practice for testing of the present invention.
Unless otherwise defined, all technical and scientific terms used herein have the same meaning as commonly understood by a person of ordinary skill in the art.

The activation of G-protein coupled receptors and subsequent activation of adenyl cyclase catalyzes the conversion of ATP to cAMP, a second messenger involved in the regulation of numerous cellular processes. Phosphodiesterases (PDEs) are a superfamily of enzymes which play the fundamental role of hydrolysing cAMP, leading to its degradation and termination of cell signal transduction. These enzymes define and regulate intracellular cAMP gradients around specific signalling complexes, with each specific isoform having unique functional roles.

Of particular interest is the PDE4 family of enzymes, which is made up of over 25 different isoforms, many of which have important, non-redundant functions. Often, the function of a particular PDE4 isoform is conferred by its unique N-terminal, which acts as a "postcode" to anchor PDE4 enzymes to discrete intracellular domains where they sculpt signal-specific cAMP gradients. PDE4s also contain a catalytic unit and regulatory domains termed "upstream conserved regions one and two" (UCR1/2) which are highly conserved throughout the isoforms. All long-form PDE4s, such as for example PDE4D7, contain UCR1, which contains a PKA motif that becomes phosphorylated during conditions of raised cAMP. Such an action serves to activate PDE4 and rapidly reduce the local concentration of cAMP. This feedback loop underpins the transient nature of cAMP signals and ensures a rapid but fleeting response to activation of Gas-coupled receptors.

Deregulated cAMP signaling is associated with various diseases including cancer, with the PDE4D sub-family of particular interest in prostate cancer (PCa). PDE4D7, a long PDE4D isoform variant, localises to the sub-plasma membrane in PCa cells and its expression inversely correlates with disease progression. Specifically, PDE4D7 mRNA is significantly downregulated from an androgen sensitive (AS) to androgen insensitive (AI) disease phenotype, implicating PDE4D7 in PCa development and progression. Furthermore, there is a significant association between PDE4D7 expression and presence of the TMPRSS2-ERG gene fusion, the most common gene rearrangement in PCa which leads to overexpression of ERG and subsequent enhanced PCa aggressiveness.

Herein the inventors describe data obtained from prostate cancer cells in which expression of PDE4D7 has been specifically reduced using shRNA mediated knockdown. Surprisingly the inventors found that re-expressing PDE4D7 in these knockdown cells reduced proliferation. Furthermore expression of PDE4D7 was able to reduce resistance to drug treatments such as antiandrogen resistance, PARP inhibitor resistance or chemotherapy resistance.

Therefore in a first aspect the invention relates to a product for use in the treatment, prevention or amelioration of prostate cancer in a subject in need thereof, wherein the product induces the expression of PDE4D7 or promotes activity of phosphodiesterase 4D7. Alternatively the invention relates to a method of treating, preventing or ameliorating prostate cancer in a subject in need thereof, the method comprising administering to the subject product induces the expression of PDE4D7 or promotes activity of phosphodiesterase 4D7.

As has been set out above, the present invention concerns in one aspect phosphodiesterase 4D7 (PDE4D7) for use as a prostate cancer marker. The term "phosphodiesterase 4D7" or "PDE4D7" relates to the splice variant 7 of the human phosphodiesterase PDE4D, i.e. the human phosphodiesterase PDE4D7 gene, preferably to the sequence as defined in Genbank Accession No: AF536976 (version AF536976.1, GI:22901883 as of 3 Mar. 2009), more preferably to the nucleotide sequence as set forth in SEQ ID NO: 1, which corresponds to the sequence of the above indicated Genbank Accession number of the PDE4D7 transcript and corresponding protein accession number AAN10118 (version AAN10118.1), and also relates to the corresponding amino acid sequence as set forth in SEQ ID NO: 2, which corresponds to the sequence of the above indicated Genbank Accession number of the PDE4D7 polypeptide encoded by the PDE4D7 transcript. The term also comprises nucleotide sequences showing a high degree of homology to PDE4D7, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 1, or amino acid sequences being at least 75%, 80%, 85%, 90%, 91%, 92%, 93%, 94%, 95%, 96%, 97%, 98% or 99% identical to the sequence as set forth in SEQ ID NO: 2, or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 2, or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1.

The term "human phosphodiesterase PDE4D7 gene", "PDE4D7 gene" or "PDE4D7 marker gene" as used herein relates to the gene encoding phosphodiesterase 4D. Preferably, the term relates to a gene expressing phosphodiesterase 4D as splice variant 7, e.g. the specific exon combination as defined in Genbank Accession No: AF536976 (version AF536976.1, GI:22901883 as of 3 Mar. 2009) or as set forth in SEQ ID NO: 1. The term also relates to DNA molecules derived from mRNA transcripts encoding phosphodiesterase 4D spliced as variant 7, preferably cDNA molecules.

The term "phosphodiesterase 4D7", "phosphodiesterase 4D7 protein", or "PDE4D7 protein" as used herein relates to the 4D7 splice variant encoded protein as defined herein above. Preferably, the term relates to a protein encoded by the phosphodiesterase 4D gene as splice variant 7, e.g. the specific exon combination as defined in Genbank Accession No: AF536976 (version AF536976.1, GI:22901883 as of 3 Mar. 2009) or as set forth in SEQ ID NO: 2. The term also relates to proteins or polypeptides derived from the protein encoded by the PDE4D7 gene and which have been modified by addition of additional amino acid(s) or other modifications such as sugar residues, tags, phosphorylation, acetylation, methylation, ubiquitination or other modifications, or has been modified by the removal of one or more amino acids such as but not limited to a signal peptide.

The present invention is prompted by the inventors finding that re-introducing PDE4D7 in PDE4D7 knockdown prostate cancer cells has several beneficial effects such as reduction of cancer cell proliferation and reversal of therapy resistance. It is envisaged that such effect can thus be established in two manners: 1) by increasing the expression of PDE4D7 protein, that is, by increasing the amount of phosphodiesterase 4D7 protein in the prostate cancer cells or indirectly by increasing the expression level of the PDE4D7 isoform of the PDE4D gene, that is, by promoting transcription of the PDE4D gene isoform 7 or by otherwise increasing the amount of PDE4D7 mRNA; or 2) by increasing the activity of PDE4D7 enzyme, for example by removing or inhibiting antagonists of the PDE4D7 protein enzymatic activity, or stimulating agonists of the PDE4D7 protein enzyme activity, or by introducing additional protein (directly or indirectly), by introducing an active form of PDE4D7 protein or by a compound directly or indirectly engaging with PDE4D7 to active it.

Therefore, the present invention broadly defines a product for inducing the expression of PDE4D7 or promoting the activity of phosphodiesterase 4D7. In an embodiment the product is a compound or composition.

When used herein the term compound may refer to a chemical compound (also referred to as "small molecule therapy"), a biological such but not limited to a polynucleotide, a polypeptide, or a sugar polymer.

When used herein the term composition may refer to a mixture of compounds or a combination of a compound with adjuvants. A composition may be a solution or a dry composition.

The inventors envision several ways to achieve increased PDE4D7 expression or phosphodiesterase 4D7 activity in a subject, some non-limiting examples are listed below, however it is understood that the invention extends to any method or compound known to the skilled person to achieve increased expression of PDE4D7 or increased activity of the phosphodiesterase 4D7 isoform. Thus in an embodiment the product is selected from:
- a gene therapy for inducing PDE4D7 expression;
- a compound that induces PDE4D7 expression;
- a compound directly stimulating or promoting phosphodiesterase 4D7 activity;
- a compound indirectly stimulating or promoting phosphodiesterase 4D7 activity;
- an inhibitor of a transcriptional inhibitor of PDE4D7;
- an inhibitor of an inhibitor of phosphodiesterase 4D7 enzymatic activity
- mRNA encoding a phosphodiesterase 4D7 protein;
- a phosphodiesterase 4D7 protein;
- a phosphodiesterase 4D7 activity promoting amyloid beta peptide.

When used herein, a gene therapy for inducing PDE4D7 expression refers to a method to introduce a nucleotide encoding the phosphodiesterase 4D7 isoform in a cell, preferably a cell of a subject having prostate cancer, more preferably in a prostate cancer cell or a metastasis thereof. The nucleotide may for example be a viral vector or a non-viral vector, however it is understood that any nucleotide which can be introduced in a cell of a patient and express PDE4D7 may be used.

Viral vectors are a known strategy for gene therapy, typically viral-vector gene therapies use modified viruses as drug-delivery vehicles to introduce specific DNA or RNA sequences into cells. The vector is packaged using the viral proteins allowing infection of cells and expression of its viral genome. Typically the viral vector is modified such that no new viral particles can be produced upon infection of the target cell. Non limiting examples include retroviruses (RV), adenoviruses (AV), adeno-associated viruses (AAV), lentiviruses (LV), and herpes simplex viruses (HSV). Other ways to use viral particles or viral vectors to introduce the PDE4D7 encoding nucleotide are known to the person skilled in the art and also envisaged to be encompassed by the invention.

Alternatively to viral vector based gene therapy, a non-viral vector may be used. These vectors typically contain a promotor to drive expression of the relevant construct (e.g. PDE4D7), and typically require some means to introduce the vector into the target cell. Means to deliver a non-viral vector to a target cell in a subject are known to the skilled person, and for example reviewed in Ramamoorth M, Narvekar A. Non viral vectors in gene therapy- an overview. J Clin Diagn Res. 2015 Jan;9(1):GE01-6 (hereby incorporated by reference in its entirety). For example, nanoparticles can be used to encapsulate the vector. Non limiting examples are lipid based nanoparticles, peptide based nanoparticles, cationic lipid based nanoparticles, apolipoprotein based nanoparticles, (synthetic) polymer based nanoparticles such as polyethylenimine (PEI), chitosan, polylactate, polylactide, polyglucoside, denriomer or polymethracylate based nanoparticles. When used herein a nanoparticle is a small particle which can be used a sa carrier to deliver a cargo (payload) in a patient. Preferably the cargo is the product as broadly defined herein. Therefore a nanoparticle can be used to deliver the product induces the expression of PDE4D7 or promotes activity of phosphodiesterase 4D7 to a site in patient, e.g. a cell, tissue or organ. Other ways to use non- viral vectors to introduce the PDE4D7 encoding nucleotide are known to the person skilled in the art and also envisaged to be encompassed by the invention.

Therefore in an embodiment the gene therapy is selected from: a viral vector capable of expressing PDE4D7 in a subject or a non-viral vector capable of expressing PDE4D7 in a subject. In an embodiment the product is delivered using a nanoparticle.

When used herein the term vector is used to indicate any particle (e.g., a plasmid, a cosmid, a Lambda phage) used as a vehicle to artificially carry a foreign nucleic sequence - usually DNA - into another cell, where it can be replicated and/or expressed.

When used herein a compound that induces PDE4D7 expression is intended to refer to any biological or chemical compound that is able to increase the expression of PDE4D7. This may for example be achieved by promoting transcription of the PDE4D7 gene or inhibiting degradation of the phosphodiesterase 4D7 isoform protein. For example the compound may engage a signal transduction pathway to indirectly promote transcription of the PDE4D7 or directly interact with the promoter region of the genomic DNA to promote transcription of the PDE4D7 isoform.

When used herein promoting transcription of PDE4D7 refers to increasing the transcription of PDE4D7 resulting in a higher amount of PDE4D7 mRNA transcripts, and/or preferably in a higher amount of phosphodiesterase 4D7 isoform protein in the cell. The promoting may be specific for PDE4D7, specific for all PDE4D isoforms, specific for all PDE4 or even all PDE family members. In other words, increasing expression of PDE4D7 does not exclude that other PDE4D isoforms, other PDE4 family members or even other PDE family members are also increased in expression.

When used herein, a compound directly stimulating or promoting phosphodiesterase 4D7 activity is intended to refer to a compound which directly engages with the phosphodiesterase 4D7 isoform protein enzymatic activity. Without wishing to be bound be theory, it is theorized that PDE proteins such as the PDE4D7 protein can exist in an active and inactive conformation, wherein activity can be induced by other compounds through interaction with the PDE protein, for example by promoting an active conformation of the protein or by blocking or preventing binding of inhibitors, or by modifying the protein to promote activity (for example by phosphorylation or other known protein modifications). When used herein, enzymatic activity when referring to a phosphodiesterase refers to catalysis of the hydrolysis of cAMP and/or cGMP.

When used herein, a compound indirectly stimulating or promoting phosphodiesterase 4D7 activity is intended to refer to a compound which indirectly engages with the phosphodiesterase 4D7 isoform protein enzymatic activity. Similarly as above it is envisaged that compounds may induce activators of PDE4D7 or block inhibitors form PDE4D7 activity and thus indirectly affect the protein's enzymatic activity. The person skilled in the art is aware of methods to determine PDE or specifically PDE4D7 activity. For example, commercial products are available to assay for PDE activity, and methods have for example been described in Blair et al. Measuring cAMP Specific Phosphodiesterase Activity: A Two-step Radioassay. Bio Protoc. 2020 Apr 5;10(7):e3581, hereby incorporated in its entirety by reference.

When used herein, an inhibitor of a transcriptional inhibitor of PDE4D7 refers to a compound capable of blocking an inhibitor of PDE4D7 gene transcription. The inbitior of PDE4D7 gene transcription may be a direct inhibitor capable of binding the genomic DNA and preventing or reducing gene transcription or an indirect inhibitor which through downstream actions reduces or inhibits transcription of the PDE4D7 gene.

When used herein an inhibitor of an inhibitor of phosphodiesterase 4D7 enzymatic activity refers to a compound that can block an inhibitor of phosphodiesterase isoform 4D7 protein activity. For example the compound may function by degrading the inhibitor, preventing the inhibitor from engaging with PDE4D7 or by inhibiting the activity of the inhibitor.

When used herein, mRNA encoding a phosphodiesterase 4D7 protein refers to a RNA molecule from which the protein corresponding to SEQ ID NO: 2 or a substantially similar protein can be translated. The mRNA molecule generally comprises non translated elements such as 5' and 3' UTRs. It is anticipated that by direct introduction of PDE4D7 mRNA into the target cell (e.g. a prostate cancer cell in a subject) PDE4D7 can by upregulated. Method of delivering mRNA to a target cell are known to the skilled person, for example using nanobodies as described above.

When used herein a phosphodiesterase 4D7 protein refers to a compound comprising PDE4D7 protein or a biosimilar, or a constitutively active variant thereof, having phosphodiesterase activity. It is anticipated that by direct introduction of PDE4D7 protein into the target cell (e.g. a prostate cancer cell in a subject) PDE4D7 activity can be increased. Method of delivering protein to a target cell are known to the skilled person, for example using nanobodies as described above.

When used herein, a phosphodiesterase 4D7 activity promoting peptide refers to a peptide that is able to increase activity of the PDE4D7 enzyme. Non limiting examples are Amyloid beta (Abeta) peptides, which have been demonstrated to specifically increase long PDE4D isoforms such as PDE4D7. When used herein Amyloid beta, also known as Aβ or Abeta, refers to peptides of 37-49 amino acids (Chen et al. Acta Pharmacol Sin 38, 1205-1235 (2017)) that are the main component of the amyloid plaques found in the brains of people with Alzheimer's disease. Amyloid beta peptide (Aβ) is produced through the proteolytic processing of a transmembrane protein, amyloid precursor protein (APP), by β-and γ-secretases.

Therefore the phosphodiesterase 4D7 activity promoting peptide is preferably a Abeta peptide or derivative thereof. When used herein a Abeta peptide or derivative thereof is characterized as a peptide having a length of 16 to 50 amino acids and comprising an amino acid sequence 90%, 91%, 92%, 93%, 94%, 95%, 96%, 97%, 98%, 99% or 100% identical to the sequence defined by SEQ ID NO: 24 below (Abeta core peptide 1), more preferably comprising an amino acid sequence 90%, 91%, 92%, 93%, 94%, 95%, 96%, 97%, 98%, 99% or 100% identical to the sequence defined by SEQ ID NO: 25 below (Abeta core peptide 2). Non limiting examples are provided by Abeta 42 (SEQ ID NO: 26) and Abeta 40 (SEQ ID NO: 27). Therefore in an embodiment the Abeta peptide or derivative thereof is characterized as a peptide having a length of 42 to 50 amino acids and comprising an amino acid sequence 90%, 91%, 92%, 93%, 94%, 95%, 96%, 97%, 98%, 99% or 100% identical to the sequence defined by SEQ ID NO: 26, or a peptide having a length of 40 to 50 amino acids and comprising an amino acid sequence 90%, 91%, 92%, 93%, 94%, 95%, 96%, 97%, 98%, 99% or 100% identical to the sequence defined by SEQ ID NO: 27.
SEQ ID NO: 24 Abeta core peptide 2
   HDSGYEVHHQKLVFFAEDVGSNKGAIIG
SEQ ID NO: 25 Abeta core peptide 2
   FRHDSGYEVHHQKLVFFAEDVGSNKGAIIGLMV
SEQ ID NO: 26 Abeta 42
   DAEFRHDSGYEVHHQKLVFFAEDVGSNKGAIIGLMVGGVVIA
SEQ ID NO: 27 Abeta 40
   DAEFRHDSGYEVHHQKLVFFAEDVGSNKGAIIGLMVGGVV

The inventors describe herein several advantages for a treatment based on increasing PDE4D7 expression or activating phosphodiesterase 4D7 in a cell, such as reducing cancer cell proliferation. Moreover the inventors provide experimental data strongly suggesting that increasing PDE4D7 expression or activating phosphodiesterase 4D7 in a cell may help to overcome therapy resistance, or at least makes therapy resistant tumor cells more responsive to therapeutics, such as, among others, antiandrogens, PARP inhibitors and taxanes. Therefore in an embodiment the treatment, prevention or amelioration comprises reducing a therapy resistance.

When used herein reducing therapy resistance refers reducing or avoiding resistance to said therapy which has developed or may develop in a cancer cell. As such, co-adminstering the compound (product) that induces the expression of PDE4D7 or promotes activity of phosphodiesterase 4D7 together with a therapy will unexpectedly increase the effectiveness of the therapy, in addition to the beneficial effects observed from the compounds derscibed herein.

Therefore in an embodiment the therapy resistance is antiandrogen resistance. Antiandrogens may be androgen receptor antagonists. Non limiting examples of steroidal antiandrogens comprise 17α-Hydroxyprogesterone derivatives such as, Chlormadinone acetate, Cyproterone acetate, Megestrol acetate, Osaterone acetate; 19-Norprogesterone derivatives such as Nomegestrol acetate; 19-Nortestosterone derivatives such as Dienogest, Oxendolone; 17α-Spirolactone derivatives such as Drospirenone, Spironolactone, Others such as Medrogestone. Non-limiting examples of nonsteroidal antiandrogens comprise Bicalutamide, Flutamide, Nilutamide, Apalutamide, Darolutamide, Enzalutamide, Proxalutamide, Cimetidine and Topilutamide.

Antiandrogens may be androgen synthesis inhibitors. Non-limiting examples of CYP17A1 inhibitors such as Abiraterone acetate, Ketoconazole, Seviteronel; CYP11A1 (P450scc) inhibitors such as Aminoglutethimide; 5α-Reductase inhibitors such as Alfatradiol, Dutasteride, Epristeride, Finasteride, Saw palmetto extract.

Antiandrogens may be antigonadotropins. Non-limiting exmaples are estrogens such as estradiol (and its esters), ethinylestradiol, conjugated estrogens, diethylstilbestrol; GnRH analogues such as GnRH agonists like goserelin, leuprorelin, GnRH antagonists like cetrorelix; Progestogens such as chlormadinone acetate, cyproterone acetate, gestonorone caproate, medroxyprogesterone acetate, megestrol acetate.

Therefore in an embodiment the therapy resistance is resistance to an androgen receptor antagonist, an androgen synthesis inhibitor or an antigonadotropin, preferably an androgen receptor antagonist, an androgen synthesis inhibitor or an antigonadotropin as listed above.

In a further preferred embodiment the therapy resistance is resistance to Enzalutamide, Flutamide, Nilutamide, Bicalutamide, Topilutamide, Apalutamide, Darolutamide, Cimetidine, ketoconazole, Proxalutamide (GT-0918), Seviteronel (VT-464), or Abiraterone. In a further preferred embodiment the therapy resistance is resistance to Enzalutamide.

In an embodiment the therapy resistance is PARP inhibitor resistance. The PARP inhibitor resistance may be resistance to Olaparib, Rucaparib, Niraparib, Talazoparib, Veliparib, Pamiparib (BGB-290), Rucaparib, CEP 9722, E7016, Iniparib or 3-Aminobenzamide. Therefore in an embodiment the therapy resistance is resistance to a PARP inhibitor as listed above. In an embodiment the therapy resistance is resistance to a PARP inhibitor selected from Olaparib, Rucaparib, Niraparib, Talazoparib, Veliparib, Pamiparib (BGB-290), Rucaparib, CEP 9722, or E7016. In an embodiment the therapy resistance is resistance to Olaparib.

In an embodiment the therapy resistance is resistance to a chemotherapeutic agent.

A chemotherapeutic agent may be an alkylating agent. Non-limiting examples of alkylating agents are Cyclophosphamide, Mechlorethamine, Chlorambucil, Melphalan, Dacarbazine, Nitrosoureas, and Temozolomide. Achemotherapeutic agent may be an anthracycline. Non-limiting examples of anthracyclines are Daunorubicin, Doxorubicin, Epirubicin., Idarubicin, Mitoxantrone, and Valrubicin. A chemotherapeutic agent may be a taxane. Non-limiting examples of taxanes are Paclitaxel, Docetaxel, Abraxane, and Taxotere. A chemotherapeutic agent may be a histone deacetylase inhibitor. Non limiting examples of histone deacetylase inhibitors are Vorinostat and Romidepsin.A chemotherapeutic agent may be a topoisomerase inhibitor. Non-limiting examples of topoisomerase inhibitors are Irinotecan, Topotecan, Etoposide, Teniposide, and Tafluposide. A chemotherapeutic agent may be a kinase inhibitor. Non-limiting examples of kinase inhibitors are Bortezomib, Erlotinib, Gefitinib, Imatinib, Vemurafenib, and Vismodegib. A chemotherapeutic agent may be a nucleotide analog or precursor analog. Non-limiting examples of nucleotide analogs or precursor analogs are Azacitidine, Azathioprine, Capecitabine, Cytarabine, Doxifluridine, Fluorouracil, Gemcitabine, Hydroxyurea, Mercaptopurine, Methotrexate, and Tioguanine. A chemotherapeutic agent may be a platinum based agent. Non-limiting examples of platinum based agents are Carboplatin, Cisplatin, and Oxaliplatin. A chemotherapeutic agent may be a retinoid. Non-limiting examples of retinoids are Tretinoin, Alitretinoin, and Bexarotene. A chemotherapeutic agent may be a vinca alkaloid or derivative. Non-limiting examples of vinca alkaloids or derivatives are Vinblastine, Vincristine, Vindesine, and Vinorelbine.

Therefore in an embodiment the therapy resistance is resistance to an a chemotherapeutic agent as listed above. In an embodiment the resistance to an a chemotherapeutic agent is resistance to Paclitaxel, Docetaxel, Abraxane, Taxotere, Cabazitaxel, Daunorubicin, Doxorubicin, Epirubicin, Idarubicin, Mitoxantrone, Valrubicin, Estramustine phosphate, Alestramustine, Atrimustine, Cytestrol acetate, Estradiol mustard, Estramustine, Estromustine, ICI-85966, or Phenestrol. In an embodiment the resistance to an a chemotherapeutic agent is resistance to Ocetaxel, Cabazitaxel, Mitoxantrone, Estramustine.

The present invention further provides that an additional therapy is administered to the subject, preferably a therapy to which resistance is avoided or reversed by the product as defined herein.

Therefore in an embodiment the treatment, prevention or amelioration further comprises administering an antiandrogen. The antiandrogen may be selected from the antiandrogens described above. In an embodiment the treatment, prevention or amelioration further comprises administering an antiandrogen selected from Enzalutamide, Flutamide, Nilutamide, Bicalutamide, Topilutamide, Apalutamide, Darolutamide, Cimetidine, ketoconazole, Proxalutamide (GT-0918), or Seviteronel (VT-464), or Abiraterone. In an embodiment the treatment, prevention or amelioration further comprises administering Enzalutamide.

Therefore the treatment, prevention or amelioration further comprises administering a PARP inhibitor. The PARP inhibitor may be selected from the PARP inhibitors described above. In an embodiment the treatment, prevention or amelioration further comprises administering a PARP inhibitor selected from Olaparib, Rucaparib, Niraparib, Talazoparib, Veliparib, Pamiparib (BGB-290), Rucaparib, CEP 9722, or E7016. In an embodiment the treatment, prevention or amelioration further comprises administering Olaparib.

Therefore in an embodiment the treatment, prevention or amelioration further comprises administering a chemotherapeutic agent. The chemotherapeutic agent may be a chemotherapeutic agent as listed above. In an embodiment the treatment, prevention or amelioration further comprises administering a chemotherapeutic agent selected from: Paclitaxel, Docetaxel, Abraxane, Taxotere, Cabazitaxel, Daunorubicin, Doxorubicin, Epirubicin, Idarubicin, Mitoxantrone, Valrubicin, Estramustine phosphate, Alestramustine, Atrimustine, Cytestrol acetate, Estradiol mustard, Estramustine, Estromustine, ICI-85966, or Phenestrol. In an embodiment the treatment, prevention or amelioration further comprises administering a chemotherapeutic agent selected from to Ocetaxel, Cabazitaxel, Mitoxantrone, Estramustine.

The invention further provides a kit of parts comprising a product induces the expression of PDE4D7 or promotes activity of phosphodiesterase 4D7 as broadly defined herein. Therefore, in an embodiment the kit comprises a compound or composition wherein the compound or composition induces the expression of PDE4D7 or promotes activity of phosphodiesterase 4D7. In an embodiment the product is selected from:
- a gene therapy for inducing PDE4D7 expression;
- a compound that induces PDE4D7 expression;
- a compound directly stimulating or promoting phosphodiesterase 4D7 activity;
- a compound indirectly stimulating or promoting phosphodiesterase 4D7 activity;
- an inhibitor of a transcriptional inhibitor of PDE4D7;
- an inhibitor of an inhibitor of phosphodiesterase 4D7 enzymatic activity
- mRNA encoding a phosphodiesterase 4D7 protein;
- a phosphodiesterase 4D7 protein;
- a phosphodiesterase 4D7 activity promoting peptide.

In an embodiment the gene therapy is selected from: a viral vector capable of expressing PDE4D7 in a subject or a non-viral vector capable of expressing PDE4D7 in a subject. In an embodiment the product is comprised in a nanoparticle.

In an embodiment the kit of parts further comprises an antiandrogen and/or a PARP inhibitor and/or a chemotherapeutic agent. In an embodiment the antiandrogen is selected from Enzalutamide, Flutamide, Nilutamide, Bicalutamide, Topilutamide, Apalutamide, Darolutamide, Cimetidine, ketoconazole, Proxalutamide (GT-0918), Seviteronel (VT-464), or Abiraterone. In an embodiment the PARP inhibitor is selected from Olaparib, Rucaparib, Niraparib, Talazoparib, Veliparib, Pamiparib (BGB-290), Rucaparib, CEP 9722, or E7016. In an embodiment the chemotherapeutic agent is selected from Paclitaxel, Docetaxel, Abraxane, Taxotere, Cabazitaxel, Daunorubicin, Doxorubicin, Epirubicin, Idarubicin, Mitoxantrone, Valrubicin, Estramustine phosphate, Alestramustine, Atrimustine, Cytestrol acetate, Estradiol mustard, Estramustine, Estromustine, ICI-85966, or Phenestrol.

In a further aspect the invention relates to the *ex vivo* or *in vitro* use of a product that induces the expression of PDE4D7 or promotes activity of phosphodiesterase 4D7as broadly defined herein to increase expression of PDE4D7 and/or activity of phosphodiesterase 4D7 in a cell.

It is contemplated that any method, use or composition described herein can be implemented with respect to any other method, use or composition described herein. Embodiments discussed in the context of methods, use and/or compositions of the invention may be employed with respect to any other method, use or composition described herein. Thus, an embodiment pertaining to one method, use or composition may be applied to other methods, uses and compositions of the invention as well.

### Examples

Having now generally described the invention, the same will be more readily understood through reference to the following examples which is provided by way of illustration and is not intended to be limiting of the present invention.

### Example 1

### Methods

### Generation of stable shRNA transduced LNCaP cell lines

Stable shRNA-mediated lentiviral transduced LNCaP cell lines were generated by AMSBIO (Table 1). Briefly, shRNA-PDE4D7 was cloned into a lentivector and delivered into LNCaP clone FGC cells (ATCC) via lentivirus (MOI=10). Cells were maintained in puromycin-supplemented medium prior to selection of stably transduced cells. A stable shRNA-PDE4D7 LNCaP cell line was then re-transduced with lentivirus delivering PDE4D7 to generate a re-introduced cell line. Scrambled-shRNA was used as a control. All cells were cultured in RPMI 1640 medium (Gibco□), supplemented with 10% (v/v) foetal bovine serum, 1% (v/v) L-Glutamine and 1% (v/v) penicillin/streptomycin, and stored within a 37°C incubator with 5% CO2.

**Table 1: LNCaP FGC cells transduced with lentiviral shRNA for different levels of PDE4D7 expression.**

| **Name** | **shRNA** |
|---|---|
| WT | - |
| SC2 | shRNA -scrambled |
| P1 | shRNA-PDE4D7 |
| P1-4D7^{+/+} | shRNA-PDE4D7 + PDE4D7^{+/+} |

### Plasmid DNA transfection

LNCaP WT and stable transduced cell lines were transiently transfected with pcDNA3.1-PDE4D7-VSV plasmid DNA construct using Lipofectamine LTX with Plus Reagent (Thermo Fisher Scientific) according to manufacturer's protocol. Cells were incubated for 24h post-transfection prior to further experiments.

### xCELLigence Real-Time Cell Analysis (RTCA)

Real-time cell growth was analysed using the xCELLigence RTCA system (Agilent), which measures real-time cell adhesion across gold microelectrode biosensors on 96 well electronic plates (E-plates) within a 37°C incubator with 5% CO2. Cellular adhesion influences electrical impedance which is converted into the cell index (CI) via RTCA software. Initial background measurements were recorded with 100 µL RPMI 1640 medium, followed by addition of 100 µL LNCaP cell suspension (10,000 cells/wells) for measurement of CI over time. Approximately 24h after seeding, cells were treated with specified concentrations of drugs. Negative controls containing either 0.1% DMSO or untreated cells were also included. CI was transformed to normalised CI via the RTCA software to timepoint of treatment.

### Western Blotting

Cells were lysed in 3T3 lysis buffer (50 mM NaCl, 50 mM NaF, 30 mM sodium pyrophosphate, 25 mM HEPES, 2.5 mM EDTA, 10% (v/v) glycerol, 1% (v/v) Triton X-100; pH 7.5) supplemented with cOmplete^{™} EDTA-free protease inhibitor cocktail (Sigma-Aldrich). Lysed cells were rotated end-over-end for 30 minutes at 4°C, then centrifuged for 10 minutes at 13,000 RPM at 4oC. Supernatants were collected and protein concentration determined via Bradford assay. Equal concentrations of protein samples were separated via SDS-polyacrylamide gel electrophoresis (SDS-PAGE) and transferred onto nitrocellulose membranes for western blot. Membranes were blocked in 5% (w/v) milk powder in TBS-T for 1h prior to primary and secondary antibody incubations. Primary antibodies used: anti-PDE4D5 1:5000 (Baillie lab), anti-PDE4D7 1:500 (Baillie lab), anti-PDE4D9 1:5000 (Baillie lab), anti-Pan-PDE4D 1:5000 (Baillie lab), anti-VSV 1:5000 (Abcam, #ab1874), anti-GAPDH 1:5000 (Abcam, #ab8245), anti-AR 1:1000 (Abcam, #), anti-PSMA 1:1000, anti-E-cadherin 1:500 (Cell Signalling, #3195S), anti-TMPRSS2 1:1000 (Abcam #ab109131), anti-NKX3.1 1:1000 (Cell Signalling, #92998), anti-PSA 1:1000 (Abcam, #ab76113), anti-AR-V7 (Cell Signalling, #68492), anti-GR (Cell Signalling, #12041T). Secondary antibodies used: IRDye^{®} 800CW Donkey-anti-Rabbit IgG 1:5000 (LI-COR, #926-32213), IRDye^{®} 680RD Donkey-anti-Goat IgG 1:5000 (LI-COR, #926-68074), IRDye 800CW Goat-anti-Human IgG 1:5000 (LI-COR, #926-32232), IRDye^{®} 680RD Donkey-anti-Mouse IgG 1:5000 (LI-COR, #925-68072).

### RNA analysis

### qRT-PCR

RNA was extracted using the RNeasy Mini Kit (QIAGEN) and one-step qRT-PCR was performed with the Superscript^{™} III Platinum^{™} One-Step qRT-PCR Kit (Thermo Fisher Scientific) as per manufacturers' protocols. Experiments were carried out using the Step-One^{™} Real-Time PCR System (Thermo Fisher Scientific) at the following parameters: One cycle of 50oC for 30 minutes and 95oC for 5 minutes, followed by 45 cycles of 95oC for 15 seconds and 60oC for 45 seconds. Primers and probes were purchased from Integrated DNA Technologies (Table 2), with 400nM primer/200nM probe and 1ng/ul RNA per reaction. Ct values were quantified using the 2-ΔΔCt method and statistical analysis was performed on ΔΔCt values.

**Table 2: Primers and probe sequences used for RT-qPCR. PUM1, TBP, ACTB and HPRT1 were used as housekeeping genes. All probes were labelled 5' 6-FAM/ZEN/3' IBFQ.**

| **Gene** | **Accession No.** | **Forward Primer (5'-3')** | **Reverse Primer (5'-3')** | **Probe (5'-3')** |
|---|---|---|---|---|
| PDE4D5 | NM_00119 7218.1 | GCTTCTCAGCAGCAA CATC (SEQ ID NO: 3) | TGCCATTGTCCACATCA AAA (SEQ ID NO: 4) | ACAGCGGCGTTTCACG GTGGCACA (SEQ ID NO: 5) |
| PDE4D7 | NM_00116 5899.1 | GAACATTCAACGACC AACCA (SEQ ID NO: 6) | TGCCATTGTCCACATCA AAA (SEQ ID NO: 7) | CTGCCGCTGATTGCTAT CACTTCTGCA (SEQ ID NO: 8) |
| PDE4D9 | NM_00119 7220.1 | ATGAGCATTATTATG AAGCCAAGATC (SEQ ID NO: 9) | GTGCCATTGTCCACATC AAAAC (SEQ ID NO: 10) | CTACAAGTTCCCTAAG GACTGCAGAGG (SEQ ID NO: 11) |
| PUM1 | NM_00102 0658.2 | GCCAGCTTGTCTTCA ATGAAAT (SEQ ID NO: 12) | CAAAGCCAGCTTCTGTT CAAG (SEQ ID NO: 13) | ATCCACCATGAGTTGG TAGGCAGC (SEQ ID NO: 14) |
| TBP | NM_00319 4.4 | GCCAAGAAGAAAGT GAACATCAT (SEQ ID NO: 15) | ATAGGGATTCCGGGAG TCAT (SEQ ID NO: 16) | TCAGAACAACAGCCTG CCACCTTA (SEQ ID NO: 17) |
| HPRT1 | NM_00019 4.2 | GAGGATTTGGAAAG GGTGTTTATT (SEQ ID NO: 18) | ACAGAGGGCTACAATG TGATG (SEQ ID NO: 19) | ACGTCTTGCTCGAGAT GTGATGAAGG (SEQ ID NO: 20) |
| TUBA1B | NM_00608 2.3 | TGACTCCTTCAACACC TTCTTC (SEQ ID NO: 21) | TGCCAGTGCGAACTTC AT (SEQ ID NO: 22) | CCGGGCTGTGTTTGTA GACTTGGA (SEQ ID NO: 23) |

### NGS RNA sequencing

100 ng of total RNA was extracted from LNCaP cell lines and analysed via next generation RNA sequencing (RNAseq) using the NovaSeq 6000 S4 system (2x150 bp read length). RNAseq reads were mapped to the human reference genome (Ensembl 96) using STAR. Transcript per million (TPM) values were analysed between WT and shRNA transduced LNCaP cell lines and converted into z-scores. RNAseq data was further analysed via iPathwayGuide (Advaita Bio) to calculate the log2 fold change of genes associated with the prostate cancer pathway.

### Immunofluorescence

LNCaP cells were seeded onto 13mm glass coverslips 24 hours prior to transfection as previously described. Cells were fixed with 4% (v/v) paraformaldehyde for 15 minutes, blocked and permeabilised with 10% donkey serum, 0.5% BSA and 0.2% Triton-X in PBS. Coverslips were incubated overnight with anti-PDE4D7 primary antibody (1:200, Baillie Lab), followed by incubation in Alexa-Fluor 488 Donkey-anti-Goat 1:500 secondary antibody (Thermo Fisher Scientific, #A-11055). Coverslips were mounted onto slides with Duolink In Situ Mounting Medium with DAPI (Sigma-Aldrich) and imaged via the ZEISS LSM 880 laser scanning microscope with 63x oil immersion objective.

### Statistical Analysis

Values are presented as mean ± standard error of mean (S.E.M) for N≥3, unless otherwise stated. Statistical analyses were performed either via one-way ANOVA + Dunnett's multiple comparison, two-way ANOVA + Sidák's multiple comparison, or via unpaired t-test with assumed Gaussian distribution. P≤0.05 was deemed significant (^{∗} = p≤0.05, ^{∗∗} = p<0.01, ^{∗∗∗} = p<0.001, ^{∗∗∗∗} = p≤0.0001). All statistical analyses and models were performed via GraphPad Prism 9.

### Results

### Generation and sequencing of stable PDE4D7-knockdown LNCaP

A stable, PDE4D7-knockdown LNCaP cell line was generated via shRNA-mediated lentiviral transduction. Several knockdown clones were analysed for PDE4D7 expression, and P1 cells revealed the most significant PDE4D7 mRNA-mediated downregulation via qPCR (Fig. 7) and was chosen alongside the WT and scrambled-shRNA LNCaP cell lines to look into the effects of PDE4D7 expression in prostate cancer. Proof of significant PDE4D7 knockdown at mRNA [Fig. 1A] and protein level [Figs. 1B] in the P1 LNCaP cell line compared to WT was obtained. The reduction in PDE4D7 protein expression was also confirmed using confocal microscopy [Fig. 1C].

Analysis of alterations in genomic profiles between WT, scrambled and P1 cells were determined via NGS RNA seq. Top differentially expressed genes [Fig. 1D] are shown as z-scores between WT and P1 cells, with many of these genes exhibiting significant downregulation in P1 LNCaPs. Advaita pathway analysis was performed on RNA seq data, and it identified that the top differentially expressed genes are implicated in PCa-related signalling pathways [Fig. 1E], with logFC downregulated most significantly for KLK3, AR, SPINT1, TMPRSS2 and NKX3.1. To confirm that the RNAseq data was reflected at the protein level, western blotting for selected proteins was performed. Highly significant downregulation of all chosen proteins in the P1 cell line [Fig. 1F] were observed in comparison to WT. Importantly, the scrambled-shRNA cell line showed no notable difference in expression of these proteins (Fig. 8).

### PDE4D7-knockdown in LNCaP cells alters phenotype and confers resistance to enzalutamide

Knockdown of PDE4D7 in LNCaP cell line P1 significantly enhanced growth characteristics of the cells. To confirm that the effect observed was due to PDE4D7-knockdown as opposed to integration of the lentivirus, xCELLigence growth assays were also performed on WT, P1 and the scrambled-shRNA knockdown (SC2) LNCaP cell lines. Real-time growth curves showed enhanced growth of P1 cells in comparison to both WT and SC2 [Fig. 2A], correlating with a significant difference in slope analysis. Importantly, no significant difference in growth characteristics were found between the WT and SC2 LNCaP cells.

Enzalutamide is an AR antagonist used in the treatment of advanced PCa (Menon and Higano, 2013). Given that significant down-regulation of PDE4D7 expression is a characteristic of androgen-insensitive prostate cancer (Henderson et al., 2014), we wanted to investigate the effects on the growth of WT and PDE4D7-silenced PCa cells following treatment with enzalutamide. To assess optimum treatment concentrations in WT and P1 LNCaP cells, a dose response experiment was performed using increasing drug concentrations and monitored via xCELLigence technology. All concentrations tested resulted in a significant decrease in WT LNCaP cell proliferation (0.1-30 µM), with 1µM and above resulting in the least growth [Fig. 2B]. In contrast, P1 LNCaP showed minimal reductions in cell proliferation upon increasing doses of enzalutamide [Fig. 2C], and even exhibited significantly enhanced growth in comparison to DMSO-control as concentration increased to 30µM. Overall, this data strongly suggests that PDE4D7-knockdown in LNCaP cells confers resistance to enzalutamide.

Given the significant difference of AR expression between WT and P1 cells [Fig. 1E and F], we decided to test expression of the most relevant AR variants (AR-V7), however results did not reveal any obvious difference in expression [Fig. 2D]. RNA seq analysis also identified CCND1 as a significantly downregulated gene in P1 cells compared to WT [Fig. 1E].

Cyclin D1 regulates androgen-dependent transcription and cell cycle progression [REF]. Given that AR is downregulated in P1 cells, it is unsurprising therefore that CCND1 is also downregulated [Fig. 2F]. Interestingly, however, cyclin B1 appears upregulated in P1 cells compared to WT [Fig. 2E]. This could represent a compensatory mechanism in the cell, with upregulated expression of other cell cycle proteins to induce the evident enhanced proliferation.

### PDE4D7-knockdown in LNCaP cells confers resistance to PARP-inhibition and is rescued upon reintroduction of PDE4D7

Olaparib is an FDA-approved Poly (ADP-ribose) polymerase (PARP) inhibitor that is effective against LNCaP cells (Feiersinger et al., 2018). Ceralasertib is an ataxia-telangiectasia mutated and Rad3-related (ATR) inhibitor (AZD6738), that has been suggested to enhance synergistic effects in combination with olaparib in mCRPC (Lloyd et al., 2020). In light of this, we wanted to determine whether PDE4D7-knockdown affected LNCaP cells response to either of these treatments. Using real-time growth analysis, LNCaP WT proliferation decreased in a dose-dependent manner with olaparib [Fig. 3A], whereas the effect on PDE4D7-deficient LNCaPs was minimal [Fig. 3B]. Evaluation of growth rate via slope analysis yielded similar data with significant reductions in WT growth at 3, 10 and 30µM olaparib, but with unexpected reduction in slope at low concentrations (0.3 and 1 µM) in cells where PDE4D7 was knocked down. It is notable, however, that cells with reduced PDE4D7 expression were resistant to olaparib at 10 and 30µM [Fig. 3B].

Cleavage of PARP is required for late-stage apoptosis, therefore analysis of PARP/cPARP ratio can be used to assess whether cells' induction of apoptosis [REF]. Here, there is a reduction in PARP/cPARP ratio between WT and P1 cells [Fig 3C].

Given that PDE4D7-knockdown LNCaPs exhibited enhanced growth compared to WT cells [Fig. 2A] and were resistant to PARP inhibition via olaparib [Fig. 3C], we wanted to determine whether we could rescue the phenotype through re-introduction of PDE4D7 into the P1 cell line. Initially, real-time growth was evaluated for P1 and P1-PDE4D7+/+ cells with significant downregulation in neoplastic growth observed upon reintroduction of PDE4D7 to P1 cells [Fig. 3D]. Furthermore, P1-PDE4D7+/+ cells were more sensitive to olaparib treatment [Fig. 3E], as was previously apparent in the WT LNCaP cells, with a statistically significant downregulation in growth recorded when compared to both olaparib-treated P1 cells, and DMSO-treated P1-PDE4D7+/+ cells. This data re-confirms the role of PDE4D7 is blunting PCa cell growth and promoting susceptibility to clinically relevant PCa chemotherapeutics.

### PDE4D7-knockdown in LNCaP cells does not affect response to ATR inhibition yet may limit docetaxel treatment response

Docetaxel is a DNA-damage inducing chemotherapy drug and we decided to utilise it to discover whether there is a difference between the ability of WT and P1 cells to repair DNA and hence survive upon treatment. Compared to DMSO, docetaxel significantly reduced growth of both WT and PDE4D7-knockdown LNCaPs at all concentrations (Figs. 4A and 4B, respectively], except for 10µM in P1 cells which appeared to have no effect. Given that 10µM was effective in WT but not P1, we investigated how PDE4D7-reintroduction to P1 cells affected this. As with olaparib [Fig. 3D], P1-PDE4D7+/+ cells were more sensitive to docetaxel treatment than P1 [Fig. 4C], suggesting that a paucity of PDE4D7 promotes the ability to repair associated DNA damage and protects against induction of cell death.

Interestingly, whilst PDE4D7-knockdown conferred resistance of LNCaP cells to Olaparib at 10-30µM, these cells were extremely sensitive to ceralasertib at the same concentrations. Growth curves for WT and PDE4D7-knockdown LNCaP treated with increasing concentrations of ceralasertib showed similar trends with respect to sensitivity to the compound [Fig. 4D and 4E, respectively]. Analysis of curve slopes between 0-30h post-treatment yielded similar results for both cell types. In both cases, the most significant growth inhibition occurred at 10-30µM treatment. These results highlight that PDE4D7 silencing confers resistance to PARP-inhibition via olaparib, however these cells are highly susceptible to ATR-inhibition via ceralasertib.

### PDE4D7-knockdown in LNCaP cells affects response to many current anti-cancer compounds

Many of the RNA seq genes identified as differentially expressed between WT and P1 cells are implicated in anti-androgen resistance. Signalling via the glucocorticoid receptor (GR) can bypass AR inhibition in CRPC patients leading to enhanced proliferation [REF]. As opposed to most differential genes which appeared to be downregulated in P1 cells, GR expression was upregulated in P1 and confirmed via western blot as significant protein upregulation [Fig. 5A]. We therefore decided to analyse the response to the GR antagonist mifepristone. Real-time growth analysis revealed that neither WT or P1 cell proliferation were reduced compared to DMSO from 0.3-10µM mifepristone treatment [Figs. 5B and 5C, respectively]. However, 30µM mifepristone successfully inhibited growth of WT LNCaPs yet at the same concentration had an opposing effect on P1 LNCaPs with significantly enhanced proliferation. Analysis of the growth curves show that for P1 LNCaPs with 30µM mifepristone [Fig. 5C: pink trace] initial growth was inhibited compared to lower concentrations, however this effect eventually wore off and the cells regained their proliferative potential.

Screening of the Prestwick library against DuCaP and DU145 PCa cell lines revealed that anti-parasitic drugs show activity against these cells [Ralf data - to include?]. Repurposing of these drugs such as mebendazole has showed potential in advanced PCa treatment in combination with docetaxel [REF]. Analysis of the effect of mebendazole on the WT and PDE4D7-knockdown LNCaPs showed significant differences between the cell lines responses. Interestingly, whilst WT cells showed significant downregulation of cell growth in a dose-dependent manner [Fig. 5D], the effect of P1 cells was far more substantial and more significant, with initiation of immediate cell death in the cells at both concentrations [Fig. 5E]. This data suggests that PDE4D7-knockdown confers sensitivity to mebendazole in comparison to WT LNCaPs.

### cAMP dynamics in response to PDE4D7 knockdown and effect of PDE4 activator

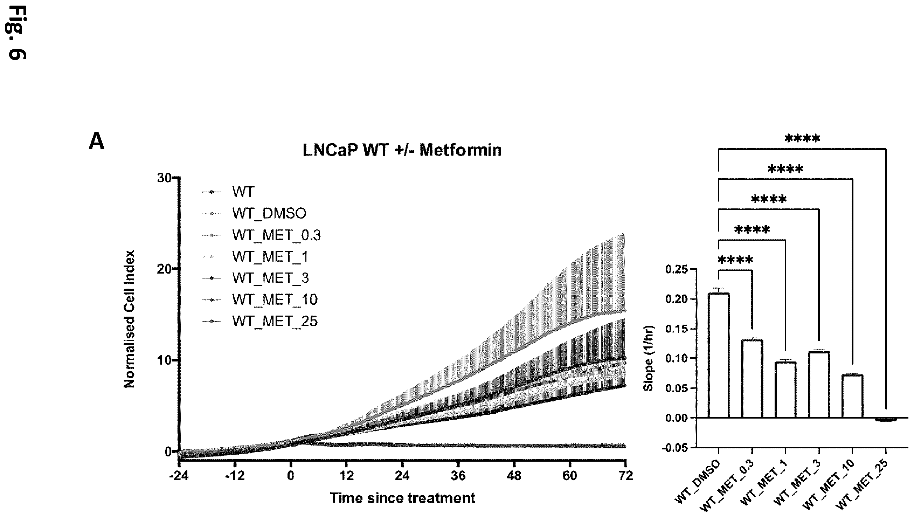

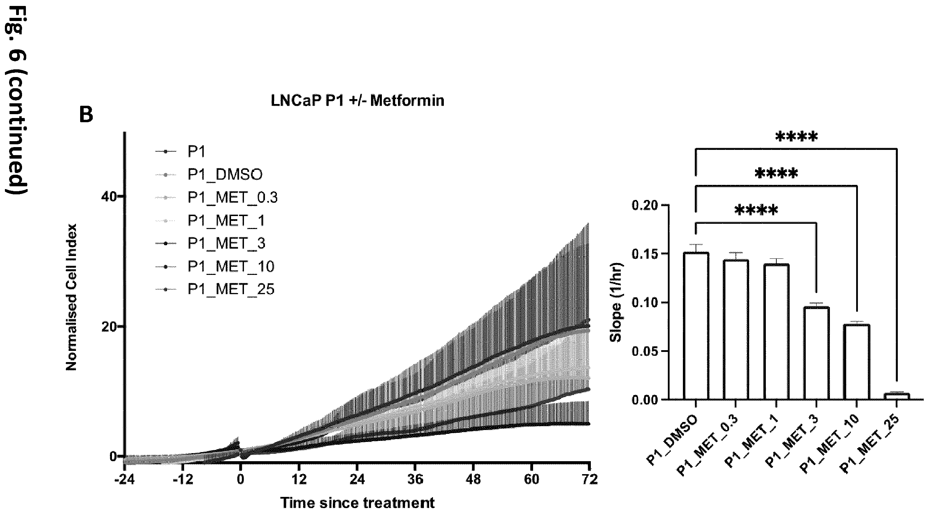

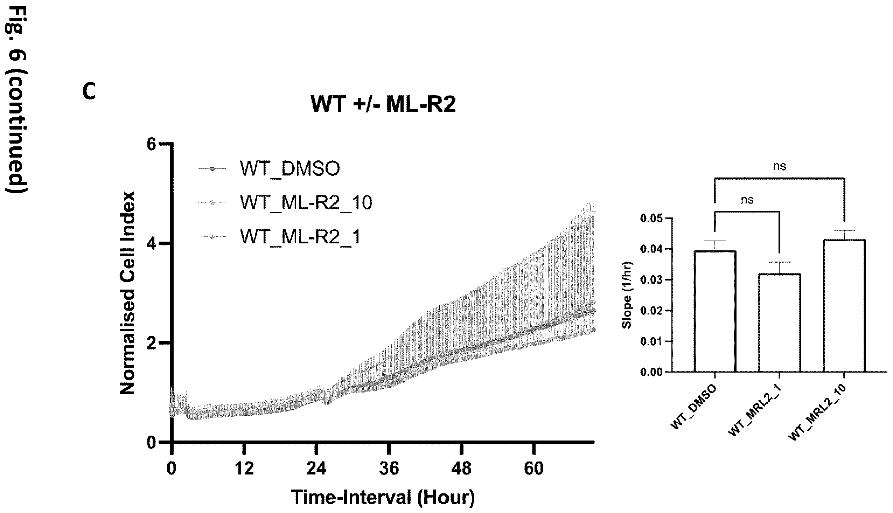

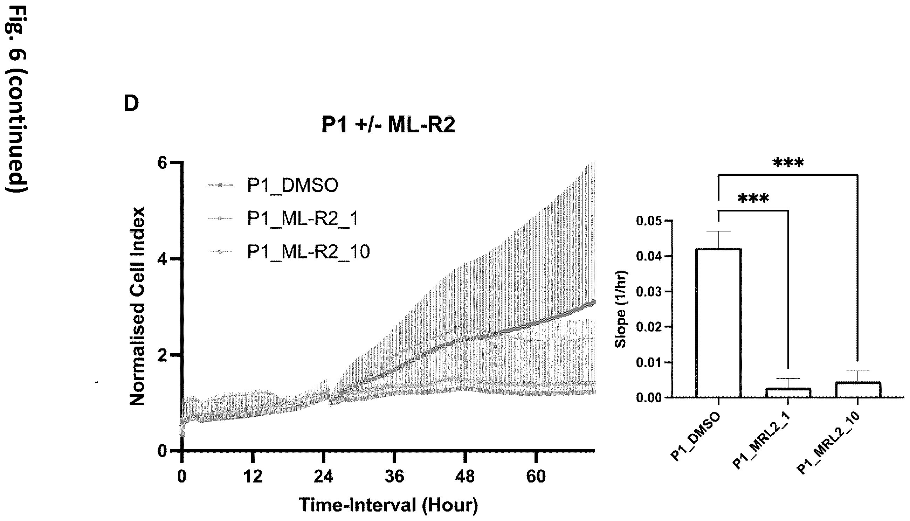

Genetically encoded cAMP-fluorescent resonance energy transfer (FRET) probes were used to investigate cAMP dynamics at subcellular regions in WT and P1 LNCaPs. CUTie cAMP FRET probes have been shown to have many advantages over conventional constructs [REF]. CUTie probes targeting the plasma-membrane (AKAP-79) or cytosol (CYTO-pDUAL) were obtained from the Zaccolo lab (REF). Treatment with rolipram induced a downregulation of FRET ratio in the membrane compartment of WT LNCaPs, indicating an increase in cAMP concentration. The effect of a combination of forskolin (adenylate cyclase activator) and IBMX (non-specific PDE inhibitor) treatment which normally gives maximal response had no further effect on these cells, highlighting that PDE4 inhibition was responsible for maximising cAMP concentration at the membrane of WT LNCaPs [Fig. 6B]. P1 LNCaPs showed no obvious difference in FRET ratio upon rolipram treatment, with a slight decrease in ratio/increase in intracellular cAMP upon forskolin/IBMX addition [Fig. 6D]. Given that PDE4D7 is known to be substantially membrane located [Fig. 1C] [REFs], inhibition via rolipram leads to upregulated cAMP at this locale in WT cells but not in P1 cells, as PDE4D7 has already been knocked down therefore basal cAMP levels are already likely to be elevated. Whilst PDE4D7 is also known to be in the cytosol, no observable difference was seen in WT and P1 LNCaPs upon rolipram treatment at this location, however forskolin/IBMX addition did appear to decrease FRET ratio/increase cAMP [Figs. 6A and 6C].

Metformin is a well-known drug used to treat type-2 diabetes, however its potential in treating numerous cancers has shown promising results [REF]. With regards to PCa it remains controversial, where it has shown to enhance overall survival/time to recurrence, however no evidence of it treating/reducing the PCa tumours itself (He et al., 2019). Given that metformin is known to decrease cAMP production (Miller et al., 2013), we decided to test its effects between WT and P1 LNCaPs. Analysis of growth phenotype shows that metformin was successful at significantly reducing growth of WT cells at all concentrations [Fig. 6E], however significant inhibition was only observed in P1 cells at highest concentrations tested [Fig. 6F].

ML-R2 is a PDE4 longform activator compound that binds allosterically to the enzyme in order to mimic PKA phosphorylation in the UCR1 domain [REF]. As PDE4D7 knockdown leads to an enhanced growth phenotype in LNCaPs, we decided to test whether this could be rescued using the PDE4 activator. Our results revealed that, whilst there was no effect on proliferation in WT LNCaPs at either concentration [Fig. 6G], ML-R2 addition caused inhibition of cell growth in P1 cells [Fig. 6H]. Whilst PDE4D7-knockdown in LNCaPs leads to enhanced cell growth, activation of PDE4 in these cells has an inhibitory effect on their proliferative potential, highlighting again the importance of the PDE4 family, specifically PDE4D7, in driving PCa.

### Example 2

Stable P1-PDE4D7+/+ LNCaP cell line were analyzed using cell index (N=3, Figure 9). Stable reintroduction resulted in expression of PDE4D7 protein as confirmed by Western blot. Stable reintroduction of PDE4D7 into P1 LNCaPs reduced real-time growth. Statistically significant downregulation of growth in P14D7+ LNCaPs was observed using cell index and slope analysis. It was confirmed that reintroduction of PDE4D7 in P1-PDE4D7 LNCaP cells reversed the knockdown phenotpy (N=3 completed just need to plot graph).

The PDE4 long form activator ML-R2 was introduced in WT, P1 and P14D7 cells and real-time growth of LNCaPs (N=3) was reviewed (Fig. 10). MLR2 has no effect on growth of WT LNCaPs, MLR2 attenuates growth of P1 LNCaPs and stable PDE4D7-reintroduction to P1 LNCaPs rescues phenotype of WT LNCaPs on MLR2 sensitivity.

P1 and P1+4D7 LNCaP cells were treated with Olaparib (N=3, Fig. 11). As a control, P1+4D7 cells were treated with DMSO. A+B) Reintroduction of PDE4D7 to P1 enhances sensitivity to Olaparib, however reintroduction of PDE4D7 alone also reduces growth to same extent.

Expression of PARP/cPARP in WT and P1 LNCaP +/- Olaparib/docetaxel treated cells was investigated (N=3, Fig. 12). Treatment with Olaparib/docetaxel increased PARP cleavage in WT LNCaPs correlating with induction of apoptosis (as seen in xCELLigence). No increase in cleaved PARP ratio in P1 LNCaPs correlating that these cells are not undergoing apoptosis in response to treatment (correlating with xCELLigence).

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

All references cited herein, including journal articles or abstracts, published or corresponding patent applications, patents, or any other references, are entirely incorporated by reference herein, including all data, tables, figures, and text presented in the cited references. Additionally, the entire contents of the references cited within the references cited herein are also entirely incorporated by references.

Reference to known method steps, conventional methods steps, known methods or conventional methods is not in any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

## Claims

1. A product for use in the treatment, prevention or amelioration of prostate cancer in a subject in need thereof, wherein the product induces the expression of PDE4D7 or promotes activity of phosphodiesterase 4D7.

2. Product for use according to claim 1, wherein the product is a compound or composition.

3. Product for use according to claim 1 or 2, wherein the product is selected from:
- a gene therapy for inducing PDE4D7 expression;
- a compound that induces PDE4D7 expression;
- a compound directly stimulating or promoting phosphodiesterase 4D7 activity;
- a compound indirectly stimulating or promoting phosphodiesterase 4D7 activity;
- an inhibitor of a transcriptional inhibitor of PDE4D7;
- an inhibitor of an inhibitor of phosphodiesterase 4D7 enzymatic activity
- mRNA encoding a phosphodiesterase 4D7 protein;
- a phosphodiesterase 4D7 protein;
- a phosphodiesterase 4D7 activity promoting peptide.

4. Product for use according to claim 3 wherein the gene therapy is selected from: a viral vector capable of expressing PDE4D7 in a subject or a non-viral vector capable of expressing PDE4D7 in a subject.

5. Product for use according to claim 3 wherein the product is delivered using a nanoparticle.

6. Product for use according to any one of claims 1 to 5 wherein the treatment, prevention or amelioration comprises reducing a therapy resistance.

7. Product for use according to claim 6 wherein the therapy resistance is antiandrogen resistance, preferably resistance to Enzalutamide, Flutamide, Nilutamide, Bicalutamide, Topilutamide, Apalutamide, Darolutamide, Cimetidine, ketoconazole, Proxalutamide (GT-0918), Seviteronel (VT-464), or Abiraterone, more preferably resistance to Enzalutamide.

8. Product for use according to claim 6 or 7 wherein the treatment, prevention or amelioration further comprises administering an antiandrogen, preferably an antiandrogen selected from Enzalutamide, Flutamide, Nilutamide, Bicalutamide, Topilutamide, Apalutamide, Darolutamide, Cimetidine, ketoconazole, Proxalutamide (GT-0918), Seviteronel (VT-464), or Abiraterone.

9. Product for use according to claim 6 wherein the therapy resistance is PARP inhibitor resistance, preferably resistance to Olaparib, Rucaparib, Niraparib, Talazoparib, Veliparib, Pamiparib (BGB-290), Rucaparib, CEP 9722, or E7016, more preferably wherein the therapy resistance is resistance to Olaparib.

10. Product for use according to claim 6 or 9 wherein the treatment, prevention or amelioration further comprises administering a PARP inhibitor to the subject, preferably a PARP inhibitor selected from Olaparib, Rucaparib, Niraparib, Talazoparib, Veliparib, Pamiparib (BGB-290), Rucaparib, CEP 9722, or E7016.

11. Product for use according to claim 6 wherein the therapy resistance is resistance to a chemotherapeutic agent, preferably resistance to a chemotherapeutic agent selected from: Paclitaxel, Docetaxel, Abraxane, Taxotere, Cabazitaxel, Daunorubicin, Doxorubicin, Epirubicin, Idarubicin, Mitoxantrone, Valrubicin, Estramustine phosphate, Alestramustine, Atrimustine, Cytestrol acetate, Estradiol mustard, Estramustine, Estromustine, ICI-85966, or Phenestrol, more preferably wherein the therapy resistance is resistance to Ocetaxel, Cabazitaxel, Mitoxantrone, Estramustine.

12. Product for use according to claim 6 or 11 wherein the treatment, prevention or amelioration further comprises administering a chemotherapeutic agent to the subject, preferably a chemotherapeutic agent selected from: Paclitaxel, Docetaxel, Abraxane, Taxotere, Cabazitaxel, Daunorubicin, Doxorubicin, Epirubicin, Idarubicin, Mitoxantrone, Valrubicin, Estramustine phosphate, Alestramustine, Atrimustine, Cytestrol acetate, Estradiol mustard, Estramustine, Estromustine, ICI-85966, or Phenestrol.

13. A kit of parts comprising a product as defined in any one of claims 1 to 5.

14. The kit of parts of claim 13 further comprising an antiandrogen and/or a PARP inhibitor and/or a chemotherapeutic agent, preferably
wherein the antiandrogen is selected from Enzalutamide, Flutamide, Nilutamide, Bicalutamide, Topilutamide, Apalutamide, Darolutamide, Cimetidine, ketoconazole, Proxalutamide (GT-0918), Seviteronel (VT-464), or Abiraterone;
wherein the PARP inhibitor is selected from Olaparib, Rucaparib, Niraparib, Talazoparib, Veliparib, Pamiparib (BGB-290), Rucaparib, CEP 9722, or E7016;
wherein the chemotherapeutic agent is selected from Paclitaxel, Docetaxel, Abraxane, Taxotere, Cabazitaxel, Daunorubicin, Doxorubicin, Epirubicin, Idarubicin, Mitoxantrone, Valrubicin, Estramustine phosphate, Alestramustine, Atrimustine, Cytestrol acetate, Estradiol mustard, Estramustine, Estromustine, ICI-85966, or Phenestrol.

15. The *ex vivo* or *in vitro* use of a product as defined in any one of claims 1 to 5 or the kit as defined in claim 13 or 14 to increase expression of PDE4D7 and/or activity of phosphodiesterase 4D7 in a cell.
